# EUROPEAN PATENT APPLICATION

(11) **EP 4 230 222 A1**
(43) Date of publication of application: **23.08.2023**
(21) Application number: 22157288.6
(22) Date of filing: 17.02.2022
(51) Int. Cl.: A61K 41/00, A61K 47/68, B82Y 5/00, A61P 35/00, A61K 9/00

(54) **COMBINATION THERAPY WITH AN ANTI-AXL ANTIBODY-PBD CONJUGATE AND NANOCUPS**

(71) Applicant: Oxsonics Limited, Oxford, OX4 4GA (GB); ADC Therapeutics SA, 1066 Epalinges (CH); MedImmune Limited, Cambridge Cambridgeshire CB21 6GH (GB)
(72) Inventor: The designation of the inventor has not yet been filed
(74) Representative: J A Kemp LLP

(57) **Abstract**

This disclosure relates to therapies in which an antibody drug conjugate is administered together with a composition comprising nanoscale cavitation initiators. Application of ultrasound following administration provides effective, targeted delivery of antibody drug conjugate to the diseased site. The therapies are useful in the treatment of solid tumour.

## Description

### Field of the invention

The invention relates to the treatment of solid tumour using a therapy comprising antibody drug conjugates and nanoscale initiators of inertial cavitation.

### Background

AXL is member of the human TAM family of receptor tyrosine kinases. The Gas6/AXL signalling pathway is associated with tumour cell growth, metastasis, invasion, epithelial-mesenchymal transition (EMT), angiogenesis, drug resistance, immune regulation and stem cell maintenance. Increased AXL expression has been associated with numerous cancers including lung cancer, breast cancer, pancreatic cancer, ovarian cancer, colon cancer and melanoma among others. Different therapeutic agents targeting AXL have been developed, typically including small molecule inhibitors, monoclonal antibodies (mAbs), nucleotide aptamers, soluble receptors, and several natural compounds.

### Summary of the Invention

We have previously developed an antibody drug conjugate comprising an anti-AXL antibody conjugated to a pyrrolobenzodiazepine (PBD) dimer. We have now found that by co-administering such a conjugate with nanoscale cavitation initiators as described herein, the delivery of the conjugate to malignant cells in a solid tumour is significantly enhanced. The nanoscale cavitation initialtors carry a stabilised, nanoscale gas bubble. On exposure to focussed therapeutic ultrasound, the bubble expands and collapses, providing inertial cavitation. This produces a mechanical streaming effect which causes significantly increased delivery of the coadministered conjugate.

Accordingly in a first aspect is provided an antibody drug conjugate for use in a method of treating a solid tumour in a subject, which method comprises administering to the subject:
(i) a pharmaceutical composition comprising a plurality of nanoparticles, each nanoparticle comprising:
   a cup having a cavity, wherein the cavity has an opening of at least 50nm in size, and
   a gas pocket present in the cavity,
   wherein the gas pocket is partially encapsulated by the cup, wherein the nanoparticle comprises a single nanocup having a single cavity, and wherein the pharmaceutical composition further comprises a pharmaceutically acceptable carrier or diluent, which is a liquid medium; and
(ii) the antibody drug conjugate, wherein the antibody drug conjugate comprises an anti-AXL antibody conjugated to a pyrrolobenzodiazepine dimer; and exposing the subject to ultrasound.

In a second aspect is provided a method of treating a solid tumour in a subject, which method comprises administering to the subject:
(i) a pharmaceutical composition comprising a plurality of nanoparticles, each nanoparticle comprising:
   a cup having a cavity, wherein the cavity has an opening of at least 50nm in size, and
   a gas pocket present in the cavity,
   wherein the gas pocket is partially encapsulated by the cup, wherein the nanoparticle comprises a single nanocup having a single cavity, and wherein the pharmaceutical composition further comprises a pharmaceutically acceptable carrier or diluent, which is a liquid medium; and (ii) an antibody drug conjugate comprising an anti-AXL antibody conjugated to a pyrrolobenzodiazepine dimer; and exposing the subject to ultrasound.

In a third aspect is provided the use of an antibody drug conjugate in the manufacture of a medicament for use in a method of treating a solid tumour in a subject, which method comprises administering to the subject:
(i) a pharmaceutical composition comprising a plurality of nanoparticles, each nanoparticle comprising:
   a cup having a cavity, wherein the cavity has an opening of at least 50nm in size, and
   a gas pocket present in the cavity,
   wherein the gas pocket is partially encapsulated by the cup, wherein the nanoparticle comprises a single nanocup having a single cavity, and wherein the pharmaceutical composition further comprises a pharmaceutically acceptable carrier or diluent, which is a liquid medium; and (ii) an antibody drug conjugate comprising an anti-AXL antibody conjugated to a pyrrolobenzodiazepine dimer; and exposing the subject to ultrasound.

### Description of the Figures

Figure 1 depicts a typical nanoparticle as described herein.
Figure 2 depicts schematically the process for producing nanoparticles as described herein.
Figures 3 to 8 show the results of the study described in the examples. Figure 3 shows tumour volumes the day before treatment.
Figure 4 shows cavitation recorded via passive acoustic mapping for animals treated with nanoparticles and exposed to ultrasound.
Figure 5 shows average tumour growth for each treated group.
Figure 6 shows individual tumour growth for the different treatment.
Figure 7A shows survival curves for all groups and figure 7B shows representative group pairs.
Figure 8 shows average mouse weight for each group treated.

### Detailed Description

### Antibody Drug Conjugates

Antibody drug conjugates for use in the methods of the present disclosure comprise an anti-AXL antibody conjugated to a cytotoxin comprising a pyrrolobenzodiazepine (PBD) dimer. The cytotoxin is connected to the antibody via one or more linkers - examples of suitable linkers are described in more detail below. In one embodiment each linker is connected through the N10 position on one of the PBD moieties conjugated to an antibody as defined below.

Various aspects and embodiments of the disclosures herein are thus suitable for use in providing a PBD compound to a preferred site in a subject. The conjugate in some embodiments preferably allows the release of an active PBD compound that does not retain any part of the linker.

Before transport or delivery into a cell, the antibody-drug conjugate (ADC) is preferably stable and remains intact, i.e. the antibody remains linked to the drug moiety. The linkers in such preferred embodiments are stable outside the target cell and may be cleaved at some efficacious rate inside the cell. An effective linker in some embodiments will: (i) maintain the specific binding properties of the antibody; (ii) allow intracellular delivery of the conjugate or drug moiety; (iii) remain stable and intact, i.e. not cleaved, until the conjugate has been delivered or transported to its targetted site; and (iv) maintain a cytotoxic, cell-killing effect or a cytostatic effect of the PBD drug moiety. Stability of the ADC may be measured by standard analytical techniques such as mass spectroscopy, HPLC, and the separation/analysis technique LC/MS.

### Anti-AXL Antibodies

AXL is a member of the human TAM family of receptor tyrosine kinases. In some embodiments, the AXL polypeptide corresponds to Genbank accession no. AAH32229, version no. AAH32229.1 GL21619004, record update date: March 6, 2012 01 :18 PM (SEQ ID NO.11).

The term "antibody" herein is used in the broadest sense and specifically covers monoclonal antibodies, including both intact antibodies and antibody fragments, so long as they exhibit the desired biological activity, for example, the ability to bind AXL. Antibodies may be murine, rat, human, humanized, chimeric, or derived from other species. An antibody includes a full-length immunoglobulin molecule or an immunologically active portion of a full-length immunoglobulin molecule, i.e., a molecule that contains an antigen binding site that immunospecifically binds an antigen of a target of interest or part thereof, such targets including but not limited to, cancer cell or cells that produce autoimmune antibodies associated with an autoimmune disease. The immunoglobulin can be of any type (e.g. IgG, IgE, IgM, IgD, and IgA), class (e.g. IgG1, IgG2, IgG3, IgG4, IgA1 and IgA2) or subclass, or allotype (e.g. human G1m1, G1m2, G1m3, non-G1m1 [that, is any allotype other than G1m1], G1m17, G2m23, G3m21, G3m28, G3m11, G3m5, G3m13, G3m14, G3m10, G3m15, G3m16, G3m6, G3m24, G3m26, G3m27, A2m1, A2m2, Km1, Km2 and Km3) of immunoglobulin molecule.

A variety of immunoglobulin variant formats are known in the art which are derived from conventional immunoglobulins, such as bispecific antibodies, scFvs, nanobodies and the like. These are all within the scope of the term "antibody" provided they retain the CDRs required for AXL binding activity.

Thus in some embodiments the antibody comprises (i) an immunoglobulin heavy chain variable region having a CDR1 region with the amino acid sequence shown in SEQ ID NO: 3, a CDR2 region with the amino acid sequence shown in SEQ ID NO: 4, and a CDR3 region with the amino acid sequence shown in SEQ ID NO: 5; and (ii) an immunoglobulin light chain variable region having a CDR1 region with the amino acid sequence shown in SEQ ID NO: 6, a CDR2 region with the amino acid sequence shown in SEQ ID NO: 7, and a CDR3 region with the amino acid sequence shown in SEQ ID NO: 8.

The CDR sequences as disclosed herein have been identified and defined using the Kabat numbering scheme (Kabat et al., U.S. Department of Health and Human Services, 1991).

In one embodiment the antibody comprises a VH domain having the sequence according to SEQ ID NO. 1. In another embodiment the antibody comprises a VL domain having the sequence according to SEQ ID NO. 2. Thus the antibody may comprise a VH domain and a VL domain where the VH comprises the sequence of SEQ ID NO.1 and the VL domain comprises the sequence of SEQ ID NO.2.

The VH and VL domain(s) in various embodiments form an antibody antigen binding site that binds AXL.

In some embodiments the antibody is an intact antibody comprising a VH domain and a VL domain, the VH and VL domains having sequences of SEQ ID NO.1 paired with SEQ ID NO.2, such as antibody 1H12 described in WO2018/146189.

Other anti-AXL antibodies include antibody 5F11 (and antibodies comprising the CDRs thereof) - described in WO2015/193420); and antibodies 107, 148 and 733 (and the variants thereof) described in WO2018/007592 (and antibodies comprising the CDRs thereof).

The specific references to the CDRs for antibodies 107, 148 and 733 in WO2018/007592 are reproduced below and incorporated by reference:
(a) a VH region comprising the CD 1, CDR2, and CDR3 sequences of SEQ ID Nos.: 36, 37, and 38, respectively; and a VL region comprising the CDR1, CDR2, and CDR3 sequences of SEQ ID Nos.: 39, GAS, and 40, respectively, [107];
(b) a VH region comprising the CDR1, CDR2, and CDR3 sequences of SEQ ID Nos.: 46, 47, and 48, respectively; and a VL region comprising the CDR1, CDR2, and CDR3 sequences of SEQ ID Nos.: 49, AAS, and 50, respectively, [148];
(c) a VH region comprising the CDR1, CDR2, and CDR3 sequences of SEQ ID Nos.: 114, 115, and 116, respectively, and a VL region comprising the CDR1, CDR2, and CDR3 sequences of SEQ ID Nos.: 117, DAS, and 118, respectively [733].

Other anti-AXL antibodies include 20G7-D9 (Leconet et al., 2017, Clin Cancer Res 23(11): 2806-2816); YW327.6S2 (Ye et al., 2010 Oncogene 29: 5254-5264) ; and DAXL-88 (Duan et al., 2019, Scand. J. Immunol. 90(2): 12777).

As used herein, "binds AXL" is used to mean the antibody binds AXL with a higher affinity than a non-specific partner such as Bovine Serum Albumin (BSA, Genbank accession no. CAA76847, version no. CAA76847.1 GI:3336842, record update date: Jan 7, 2011 02:30 PM). In some embodiments the antibody binds AXL with an association constant (Kₐ) at least 2, 3, 4, 5, 10, 20, 50, 100, 200, 500, 1000, 2000, 5000, 10⁴, 10⁵ or 10⁶-fold higher than the antibody's association constant for BSA, when measured at physiological conditions. The antibodies of the disclosure can in some embodiments bind AXL with a high affinity. For example, in some embodiments the antibody can bind AXL with a K_{D} equal to or less than about 10⁻⁶ M, such as 1 x 10⁻⁶, 10⁻⁷, 10⁻⁸, 10-⁹,10⁻¹⁰, 10⁻¹¹, 10⁻¹², 10⁻¹³ or 10⁻¹⁴.

"Antibody fragments" comprise a portion of a full length antibody, generally the antigen binding or variable region thereof. Examples of antibody fragments include Fab, Fab', F(ab')₂, and scFv fragments; diabodies; linear antibodies; fragments produced by a Fab expression library, anti-idiotypic (anti-Id) antibodies, CDR (complementary determining region), and epitope-binding fragments of any of the above which immunospecifically bind to cancer cell antigens, viral antigens or microbial antigens, single-chain antibody molecules; and multispecific antibodies formed from antibody fragments.

The term "monoclonal antibody" as used herein refers to an antibody obtained from a population of substantially homogeneous antibodies, i.e. the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by the hybridoma method or may be made by recombinant DNA methods. The monoclonal antibodies may also be isolated from phage antibody libraries or from transgenic mice carrying a fully human immunoglobulin system.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (US 4,816,567; and Morrison et al. (1984) Proc. Natl. Acad. Sci. USA, 81:6851-6855). Chimeric antibodies include "primatized" antibodies comprising variable domain antigen-binding sequences derived from a non-human primate (e.g. Old World Monkey or Ape) and human constant region sequences.

An "intact antibody" herein is one comprising VL and VH domains, as well as a light chain constant domain (CL) and heavy chain constant domains, CH1, CH2 and CH3. The constant domains may be native sequence constant domains (e.g. human native sequence constant domains) or amino acid sequence variant thereof. The intact antibody may have one or more "effector functions" which refer to those biological activities attributable to the Fc region (a native sequence Fc region or amino acid sequence variant Fc region) of an antibody. Examples of antibody effector functions include C1q binding; complement dependent cytotoxicity; Fc receptor binding; antibody-dependent cell-mediated cytotoxicity (ADCC); phagocytosis; and down regulation of cell surface receptors such as B cell receptor and BCR.

### Modification of antibodies

The antibodies disclosed herein may be modified. For example, to make them less immunogenic to a human subject. This may be achieved using any of a number of techniques familiar to the person skilled in the art. Such techniques includes humanisation to reduce the *in vivo* immunogenicity of a non-human antibody or antibody fragment. There are a range of humanisation techniques, including 'CDR grafting', 'guided selection', 'deimmunization', 'resurfacing' (also known as 'veneering'), composite antibodies', 'Human String Content Optimisation' and framework shuffling.

### PBD Cytotoxin

Pyrrolobenzodiazepines (PBDs) suitable for use in the present disclosure in some embodiments have the ability to recognise and bond to specific sequences of DNA; the preferred sequence is PuGPu. PBDs are of the general structure:

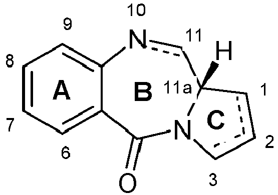

They differ in the number, type and position of substituents, in both their aromatic A rings and pyrrolo C rings, and in the degree of saturation of the C ring. In the B-ring there is either an imine (N=C), a carbinolamine(NH-CH(OH)), or a carbinolamine methyl ether (NH-CH(OMe)) at the N10-C11 position which is the electrophilic centre responsible for alkylating DNA. All of the known natural products have an (S)-configuration at the chiral C11a position which provides them with a right-handed twist when viewed from the C ring towards the A ring. This gives them the appropriate three-dimensional shape for isohelicity with the minor groove of B-form DNA, leading to a snug fit at the binding site (Kohn, In Antibiotics III. Springer-Verlag, New York, pp. 3-11 (1975); Hurley and Needham-VanDevanter, Acc. Chem. Res., 19, 230-237 (1986)). Their ability to form an adduct in the minor groove, enables them to interfere with DNA processing, hence their use as antitumour agents.

The cytotoxin is typically a PBD dimer, such as a PBD dimer, which together with the linker(s) and any optional capping group, has the following formula (II): 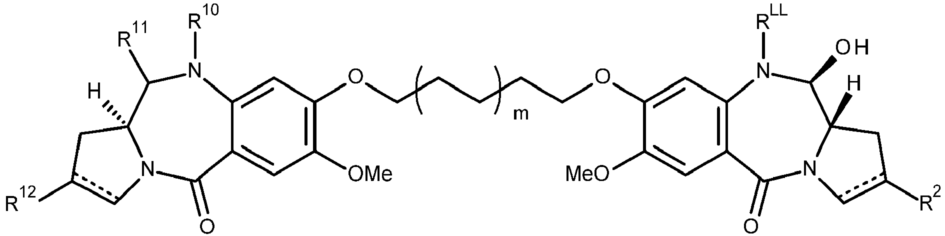 wherein R^{LL} is a linker for connection to the antibody, with examples and particular embodiments provided in more detail below in the section entitled 'Linkers'.

When released from the linker R^{LL}, the bond between N10, to which R^{LL} was originally attached, and C11 typically together form a double bond between the nitrogen and carbon atoms to which they are attached (an imine (C=N)). Similarly, in the released drug, R¹⁰ and R¹¹ typically together form a double bond between the nitrogen and carbon atoms to which they are attached. Thus the released drug may be of formula RelA: 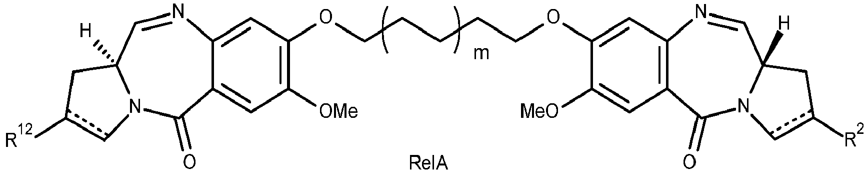 *R¹⁰ and R¹¹*

In some embodiments, R¹⁰ and R¹¹ together form a double bond between the nitrogen and carbon atoms to which they are attached.

In another embodiment, R¹⁰ is a linker R^{LLA} for connection to the antibody, Ab, and R¹¹ is OH, with R^{LLA} having the same definition as R^{LL}, and R^{LLA} and R^{LL} being the same or different.

In another embodiment, R¹⁰ is a capping group R^{C} and R¹¹ is OH.

A capping group can be used to reduce or inhibit the cytotoxic activity of the PBD, effectively forming a prodrug, but in this context is not linked to the cell binding agent. The capping group is then removed, for example in the target cell or in the tumour microenviroment, to activate the drug. Various capping groups are described in Franzyk and Christiansen, 2021, Molecules 26: 1292. (1) Prodrugs cleaved in acidic media e.g. salts of dithiocarbamates. (2) Prodrugs cleaved by reactive oxygen species. (3) Prodrugs cleaved by glutathione. (4) Prodrugs cleaved by expressed enzymes, such as oxidoreductases, hydrolases and matrix metalloproteinases. (5) Prodrugs cleaved by beta-glucuronidase, e.g. R^{C} may comprise a glucuronide, for example:

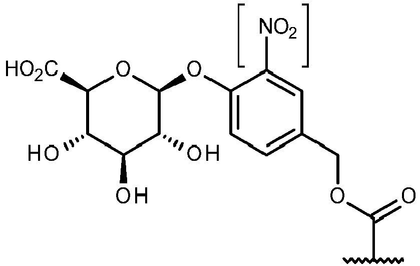

Wherein the square brackets indicate the NO₂ group is optional. In one embodiment, the NO₂ group is present.

A capping group may also be used to modify the physicochemical characteristics of the antibodody drug conjugate e.g. to make it more stable. For example, the capping group may increase the hydrophilicity of the antibody drug conjugate.

### m

In some embodiments, m is 0. In some embodiments, m is 1.

### R² and R¹²

In some embodiments, R² and R¹² are the same.

In some embodiments, there is a double bond between C2 and C3 and between C2' and C3, and R² and R¹² are both methyl.

In some embodiments, there is a single bond between C2 and C3 and between C2' and C3, and R² and R¹² are both H.

In some embodiments, there is a single bond between C2 and C3 and between C2' and C3, and R² and R¹² are both

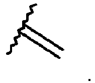

In a preferred embodiment, the drug linker, L-D is of formula (III) 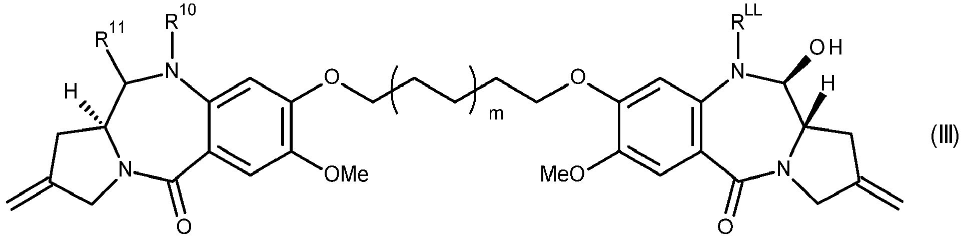 wherein R^{LL}, R¹⁰, R¹¹ and m are as defined above.

In a particular embodiment, R¹⁰, and R¹¹ together form a double bond between the nitrogen and carbon atoms to which they are attached; and m is 0 (i.e. SG2000 with a linker at N10).

In another embodiment, the drug-linker, L-D is of formula (IV): 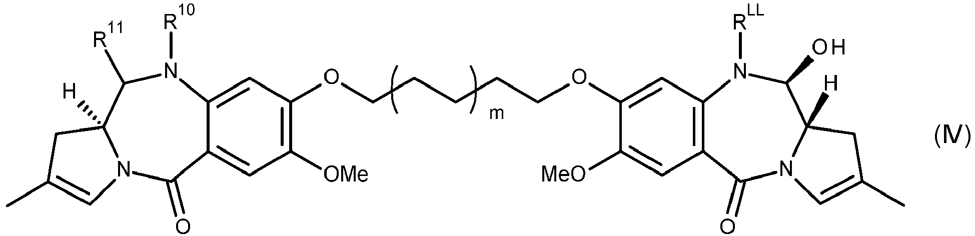 where R^{LL}, R¹⁰, R¹¹ and m are as defined above.

In a particular embodiment, R¹⁰, and R¹¹ together form a double bond between the nitrogen and carbon atoms to which they are attached; and m is 1 (i.e. SG3199 with a linker at N10).

### Drug-linkers

A wide variety of linker technologies are available in the art to link cytotoxins to cell binding agents. Linkers can incorporate various different moieties to assist with antibody-drug conjugate stability and determine drug release characteristics. For example the linker may include a cleavable moiety, such as one that is cleavable by cathepsin B (e.g. Valine-Alanine or Valine-Citrulline). Another strategy is to use a pH-sensitive linker whereby the lower pH of the endosome and lysosome compartments the hydrolysis of an acid-labile group within the linker, such as a hydrazone. Alternative a linker may be non-cleavable, which can avoid or reduce off-target effects and improve plasma stability during circulation.

The functionality that allows conjugation to the cell binding agent is based on the site of conjugation and its chemistry. N-hydroxysuccinimide esters are a common choice for functionalizing amines, especially when coupling to ε-lysine residues. For conjugation to cysteines, thiol-reactive maleimide is the most applied reactivehandle, although it is also possible to create a disulfide bridge by oxidation with a linker bearing a sulfhydryl group. Aldehyde or keto functional groups such as oxidized sugar groups or pAcPhe unnatural amino acids can be reacted with hydrazides and alkoxyamines to yield acid-labile hydrazones or oxime bonds. In addition, a hydrazine can be coupled with an aldehyde via HIPS ligation to generate a stable C-C linkage.

More recent approaches have been based on the N-linked glycosylation site in antibodies, such as Asn-297 in IgG molecules. GlycoConnect^{™} (Synaffix), using enzymes to trim the N-linked glycans to a GlcNAc core and then a further enzymatic process to introduce an activated moiety comprising azide which can then be used to incorporate the drug-linker using copper-free click chemistry. Other aspects of linker chemistry include spacers and/or moieties which mask the hydrophobicity of the cytotoxin payload, reduce cellular efflux mechanisms and/or increase overall stability, such as a polyethylene glycol (PEG) chain within the linker or a polar functional group such as a sulphonyl.

In some embodiments, the antibody drug conjugates of the disclosure can be described as Ab-L-D, where Ab is the anti-AXL antibody, D is the PBD-containing cytotoxin and L is a linker. The number of Drug moieties per Ab (the drug loading, p) depends on the number of linkers attached to each Ab, and the number of Drug moieties per linker. Typically the drug loading, p, is from 1 to 8, such as from 1 to 4 or 2 to 4. In some embodiments one Drug moiety is joined to each linker whereas in others, more than one Drug moiety may be joined to each linker (e.g. a branched linker). Drug loading is typically considered on an average basis since variations can arise from the conjugation process. Methods for determining average drug loading are known in the art.

In one embodiment the linker (e.g. shown as R^{LL} in formula (II) and (III)) is of formula (IIa): 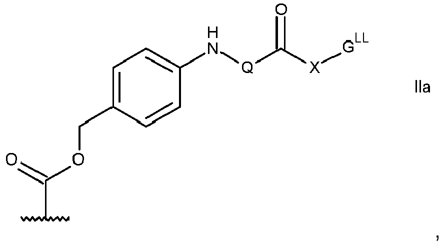 wherein
Q is: 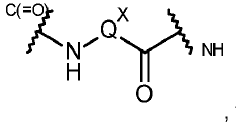 where Q^{x} is such that Q is an amino-acid residue, a dipeptide residue, a tripeptide residue or a tetrapeptide residue;
X is: 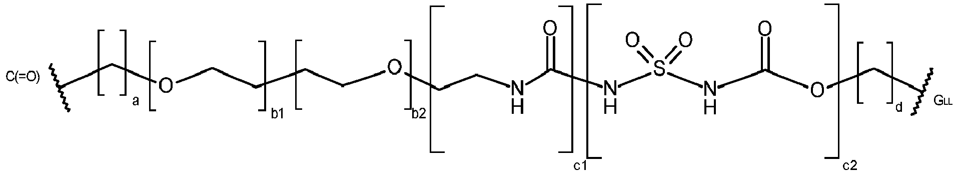 where a = 0 to 5, b1 = 0 to 16, b2 = 0 to 16, c1 = 0 or 1, c2 = 0 or 1, d = 0 to 5, wherein at least b1 or b2 = 0 (i.e. only one of b1 and b2 may not be 0) and at least c1 or c2 = 0 (i.e. only one of c1 and c2 may not be 0); and
G^{LL} is a linker group connected to Ab;

### Q^{X}

In one embodiment, Q is an amino acid residue. The amino acid may be a natural amino acid or a non-natural amino acid.

In one embodiment, Q is selected from: Phe, Lys, Val, Ala, Cit, Leu, Ile, Arg, and Trp, where Cit is citrulline.

In one embodiment, Q comprises a dipeptide residue. The amino acids in the dipeptide may be any combination of natural amino acids and non-natural amino acids. In some embodiments, the dipeptide comprises natural amino acids. Where the linker is a cathepsin labile linker, the dipeptide is the site of action for cathepsin-mediated cleavage. The dipeptide then is a recognition site for cathepsin.

In one embodiment, Q is selected from:
^{NH} -Phe-Lys-^{C=O},
^{NH} -Val-Ala- ^{C=O},
^{NH} -Val-Lys- ^{C=O},
^{NH} -Ala-Lys- ^{C=O},
^{NH}-Val-Cit- ^{C=O},
^{NH}-Phe-Cit- ^{C=O},
^{NH}-Leu-Cit- ^{C=O},
^{NH}-Ile-Cit- ^{C=O},
^{NH}-Phe-Arg- ^{C=O},
^{NH}-Trp-Cit- ^{C=O}, and
^{NH} -Gly-Val- ^{C=O};
where Cit is citrulline.

Preferably, Q is selected from:
^{NH}-Phe-Lys- ^{C=O},
^{NH}-Val-Ala- ^{C=O},
^{NH}-Val-Lys- ^{C=O},
^{NH}-Ala-Lys-^{C=O}, and
^{NH}-Val-Cit- ^{C=O}.

Most preferably, Q is selected from ^{NH}-Phe-Lys-^{C=O}, ^{NH}-Val-Cit-^{C=O} or ^{NH}-Val-Ala-^{C=O}.

Other dipeptide combinations of interest include:
^{NH}-Gly-Gly- ^{C=O},
^{NH}-Gly-Val-^{C=O}
^{NH}-Pro-Pro-^{C=O}, and
^{NH}-Val-Glu-^{C=O}.

Other dipeptide combinations may be used, including those described by Dubowchik et al., Bioconjugate Chemistry, 2002, 13,855-869, which is incorporated herein by reference.

In some embodiments, Q is a tripeptide residue. The amino acids in the tripeptide may be any combination of natural amino acids and non-natural amino acids. In some embodiments, the tripeptide comprises natural amino acids. Where the linker is a cathepsin labile linker, the tripeptide is the site of action for cathepsin-mediated cleavage. The tripeptide then is a recognition site for cathepsin. Tripeptide linkers of particular interest are:
^{NH}-Glu-Val-Ala-^{C=O}
^{NH}-Glu-Val-Cit-^{C=O}
^{NH}-αGlu-Val-Ala-^{C=O}
^{NH}-αGlu-Val-Cit-^{C=O}

In some embodiments, Q is a tetrapeptide residue. The amino acids in the tetrapeptide may be any combination of natural amino acids and non-natural amino acids. In some embodiments, the tetrapeptide comprises natural amino acids. Where the linker is a cathepsin labile linker, the tetrapeptide is the site of action for cathepsin-mediated cleavage. The tetrapeptide then is a recognition site for cathepsin. Tetrapeptide linkers of particular interest are:
^{NH}-Gly-Gly-Phe-Gly^{C=O}; (SEQ ID NO:12); and
^{NH}-Gly-Phe-Gly-Gly^{C=O} (SEQ ID NO:13).

In some embodiments, the tetrapeptide is:
^{NH}-Gly-Gly-Phe-Gly^{C=O}.

In the above representations of peptide residues, NH- represents the N-terminus, and -C=O represents the C-terminus of the residue. The C-terminus binds to the NH attached to the benzene ring.

Glu represents the residue of glutamic acid, i.e.:

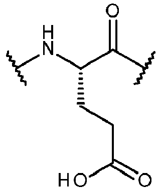

aGlu represents the residue of glutamic acid when bound via the α-chain, i.e.:

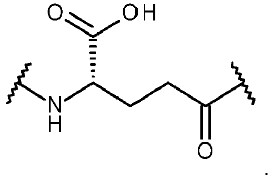

In one embodiment, the amino acid side chain is chemically protected, where appropriate. The side chain protecting group may be a group as discussed above. Protected amino acid sequences are cleavable by enzymes. For example, a dipeptide sequence comprising a Boc side chain-protected Lys residue is cleavable by cathepsin.

Protecting groups for the side chains of amino acids are well known in the art and are described in the Novabiochem Catalog, and as described above.

### G^{LL}

G^{LL} may be selected from:

| | | | |
|---|---|---|---|
| (G^{LL1-1}) | 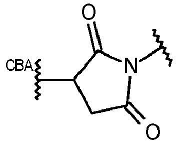 | (G^{LL8-1}) | 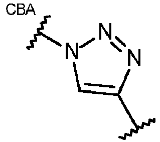 |
| (G^{LL1-2}) | 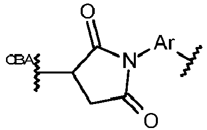 | (G^{LL8-2}) | 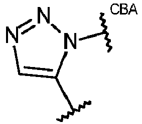 |
| (G^{LL2}) | 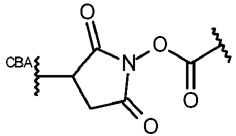 | (G^{LL9-1}) | 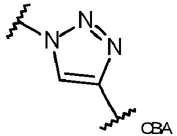 |
| (G^{LL3-1}) | 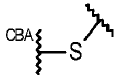 | (G^{LL9-2}) | 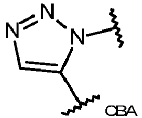 |
| (G^{LL3-2}) | 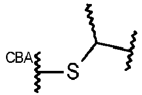 | (G^{LL10}) | 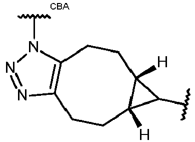 |
| (G^{LL-4}) | 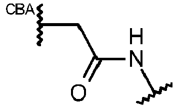 | (G^{LL11}) | 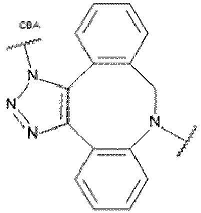 |
| (G^{LL5}) | 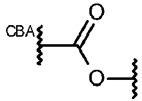 | (G^{LL12}) | 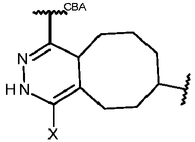 |
| (G^{LL6}) | 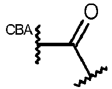 | (G^{LL13}) | 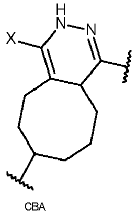 |
| (G^{LL7}) | 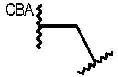 | (G^{LL14}) | 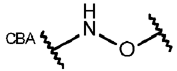 |

where Ar represents a C₅₋₆ arylene group, e.g. phenylene and X represents C₁₋₄ alkyl. CBA indicates the end of G^{LL} connected to the antibody.

In some embodiments, G^{LL} is selected from G^{LL1-1} and G^{LL1-2}. In some of these embodiments, G^{LL} is G^{LL1-1}.

In other embodiments, G^{LL} is selected from G^{LL10} and G^{LL11}. In some of these embodiments, G^{LL} is G^{LL10}.

C₅₋₆ arylene: The term "C₅₋₆ arylene", as used herein, pertains to a divalent moiety obtained by removing two hydrogen atoms from an aromatic ring atom of an aromatic compound.

In this context, the prefixes (e.g. C₅₋₆) denote the number of ring atoms, or range of number of ring atoms, whether carbon atoms or heteroatoms.

The ring atoms may be all carbon atoms, as in "carboarylene groups", in which case the group is phenylene (C₆).

Alternatively, the ring atoms may include one or more heteroatoms, as in "heteroarylene groups". Examples of heteroarylene groups include, but are not limited to, those derived from:
N₁: pyrrole (azole) (C₅), pyridine (azine) (C₆);
O₁: furan (oxole) (C₅);
S₁: thiophene (thiole) (C₅);
N₁O₁: oxazole (C₅), isoxazole (C₅), isoxazine (C₆);
N₂O₁: oxadiazole (furazan) (C₅);
N₃O₁: oxatriazole (C₅);
N₁S₁: thiazole (C₅), isothiazole (C₅);
N₂: imidazole (1,3-diazole) (C₅), pyrazole (1,2-diazole) (C₅), pyridazine (1,2-diazine) (C₆), pyrimidine (1,3-diazine) (C₆) (e.g., cytosine, thymine, uracil), pyrazine (1,4-diazine) (C₆); and
N₃: triazole (C₅), triazine (C₆).

C₁₋₄ alkyl: The term "C₁₋₄ alkyl" as used herein, pertains to a monovalent moiety obtained by removing a hydrogen atom from a carbon atom of a hydrocarbon compound having from 1 to 4 carbon atoms, which may be aliphatic or alicyclic, and which may be saturated or unsaturated (e.g. partially unsaturated, fully unsaturated). The term "C₁₋ₙ alkyl" as used herein, pertains to a monovalent moiety obtained by removing a hydrogen atom from a carbon atom of a hydrocarbon compound having from 1 to n carbon atoms, which may be aliphatic or alicyclic, and which may be saturated or unsaturated (e.g. partially unsaturated, fully unsaturated). Thus, the term "alkyl" includes the sub-classes alkenyl, alkynyl, cycloalkyl, etc., discussed below.

### X

X is: 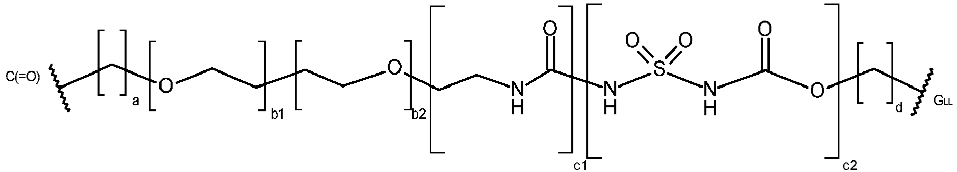 where a = 0 to 5, b1 = 0 to 16, b2 = 0 to 16, c1 = 0 or 1, c2 = 0 or 1, d = 0 to 5, wherein at least b1 or b2 = 0 and at least c1 or c2 = 0.
a may be 0, 1, 2, 3, 4 or 5. In some embodiments, a is 0 to 3. In some of these embodiments, a is 0 or 1. In further embodiments, a is 0. In further embodiments, a is 1.
b1 may be 0, 1, 2, 3, 4, 5, 6, 7, 8, 9, 10, 11, 12, 13, 14, 15 or 16. In some embodiments, b1 is 0 to 12. In some of these embodiments, b1 is 0 to 8, and may be 0, 2, 3, 4, 5 or 8. In further embodiments, b1 is 2.
b2 may be 0, 1, 2, 3, 4, 5, 6, 7, 8, 9, 10, 11, 12, 13, 14, 15 or 16. In some embodiments, b2 is 0 to 12. In some of these embodiments, b2 is 0 to 8, and may be 0, 2, 3, 4, 5 or 8. In further embodiments, b2 is 8.

Only one of b1 and b2 may not be 0.

c1 may be 0 or 1.

c2 may be 0 or 1.

Only one of c1 and c2 may not be 0.

d may be 0, 1, 2, 3, 4 or 5. In some embodiments, d is 0 to 3. In some of these embodiments, d is 1 or 2. In further embodiments, d is 2. In further embodiments, d is 5.

In some embodiments of X, a is 0, b1 is 0, c1 is 1, c2 is 0 and d is 2, and b2 may be from 0 to 8. In some of these embodiments, b2 is 0, 2, 3, 4, 5 or 8. In further embodiments, b2 is 8.

In some embodiments of X, a is 1, b2 is 0, c1 is 0, c2 is 1, d is 2, and b1 may be from 0 to 8. In some of these embodiments, b1 is 0, 2, 3, 4, 5 or 8. In further embodiments, b1 is 2.

The linker is typically connect to the PBD dimer via the N10 position, such as is shown in the location of R^{LL} in the example embodiments below.

### Drug Linker embodiments

In some embodiments, the drug-linker, L-D, is selected from:

| | |
|---|---|
| A1 | 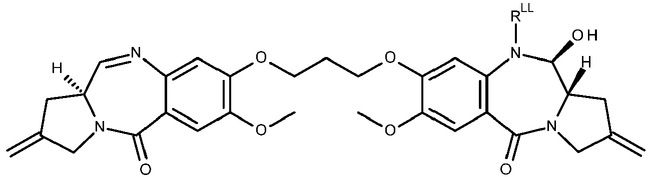 |
| A2 | 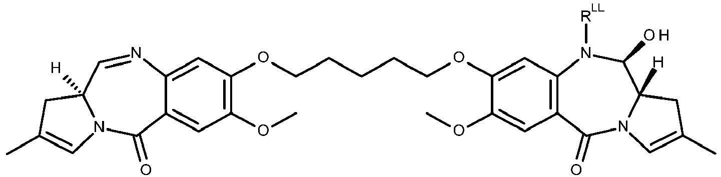 |
| A3 | 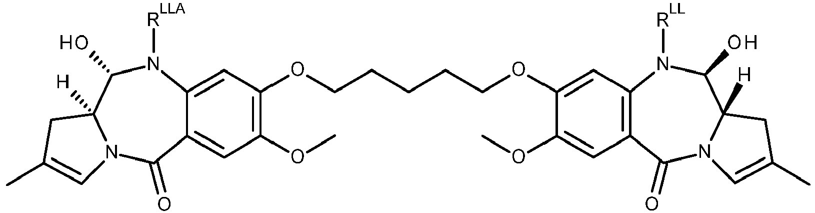 |

where R^{LL} and R^{LLA} are as described above.

In some embodiments, L-D is selected from:

| | |
|---|---|
| B1 | 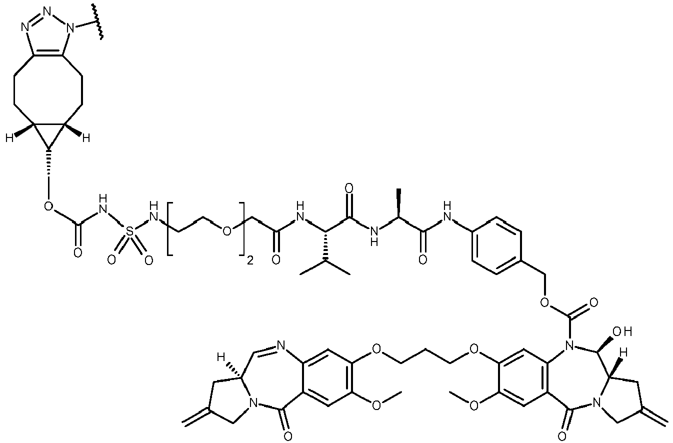 |
| B2 | 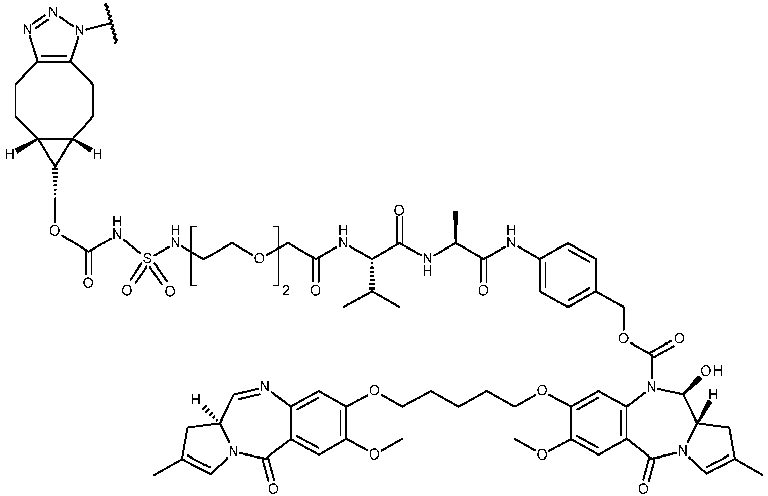 |
| B3 | 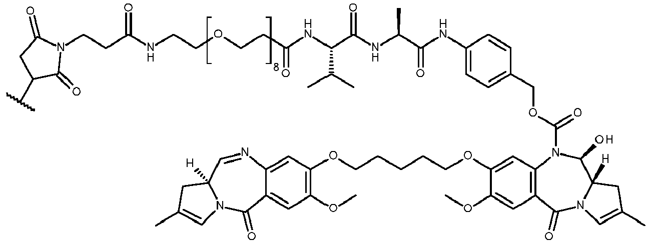 |
| B4 | 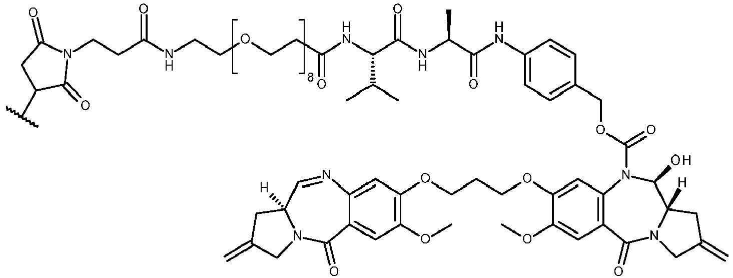 |

### Site of Conjugation and Drug Loading

Drug-linkers can be conjugated to a cell binding agent, such as an antibody, using a variety of methods known in the art and at a number of different sites. Conjugation sites include cysteine residues and lysine residues in the antibody sequence (endogenous or engineered), as well as sites of N-linked glycosylation following trimming (e.g. the GlycoConnect^{™} or GlyClick^{™} approaches). Thus in one embodiment the drug-linker is conjugated via a trimmed Asn-GlcNAc residue, typically at the endogenous N-linked glycosylation site in the antibody (Asn-297). With respect to cysteine conjugation, in one embodiment the cysteine is an endogenous cysteine located in the hinge region or Fc domain. In another embodiment the cysteine in an engineered cysteine introduced in the hinge region or Fc domain.

The drug loading is the average number of PBD drugs per cell binding agent, e.g. antibody.

The average number of drugs per antibody in preparations of ADC from conjugation reactions may be characterized by conventional means such as UV, reverse phase HPLC, HIC, mass

spectroscopy, ELISA assay, and electrophoresis. The quantitative distribution of ADC in terms of p may also be determined. By ELISA, the averaged value of p in a particular preparation of ADC may be determined (Hamblett et al (2004) Clin. Cancer Res. 10:7063-7070; Sanderson et al (2005) Clin. Cancer Res. 11:843-852). However, the distribution of p (drug) values is not discernible by the antibody-antigen binding and detection limitation of ELISA. Also, ELISA assay for detection of antibody-drug conjugates does not determine where the drug moieties are attached to the antibody, such as the heavy chain or light chain fragments, or the particular amino acid residues. In some instances, separation, purification, and characterization of homogeneous ADC where p is a certain value from ADC with other drug loadings may be achieved by means such as reverse phase HPLC or electrophoresis. Such techniques are also applicable to other types of conjugates.

For some antibody-drug conjugates, p may be limited by the number of attachment sites on the antibody. For example, an antibody may have only one or several cysteine thiol groups, or may have only one or several sufficiently reactive thiol groups through which a linker may be attached. Higher drug loading, e.g. p >5, may cause aggregation, insolubility, toxicity, or loss of cellular permeability of certain antibody-drug conjugates.

Typically, fewer than the theoretical maximum of drug moieties are conjugated to an antibody during a conjugation reaction. An antibody may contain, for example, many lysine residues that do not react with the drug-linker intermediate or linker reagent. Only the most reactive lysine groups may react with an amine-reactive linker reagent. Also, only the most reactive cysteine thiol groups may react with a thiol-reactive linker reagent. Generally, antibodies do not contain many, if any, free and reactive cysteine thiol groups which may be linked to a drug moiety. Most cysteine thiol residues in the antibodies of the compounds exist as disulfide bridges and must be reduced with a reducing agent such as dithiothreitol (DTT) or TCEP, under partial or total reducing conditions. The loading (drug/antibody ratio) of an ADC may be controlled in several different manners, including: (i) limiting the molar excess of drug-linker intermediate or linker reagent relative to antibody, (ii) limiting the conjugation reaction time or temperature, and (iii) partial or limiting reductive conditions for cysteine thiol modification.

Certain antibodies have reducible interchain disulfides, i.e. cysteine bridges. Antibodies may be made reactive for conjugation with linker reagents by treatment with a reducing agent such as DTT (dithiothreitol). Each cysteine bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol. Reactive thiol groups may be introduced into the antibody (or fragment thereof) by engineering one, two, three, four, or more cysteine residues (e.g., preparing mutant antibodies comprising one or more non-native cysteine amino acid residues). US 7521541 teaches engineering antibodies by introduction of reactive cysteine amino acids.

Cysteine amino acids may be engineered at reactive sites in an antibody, and which do not form intrachain or intermolecular disulfide linkages (Junutula, et al., 2008b Nature Biotech., 26(8):925-932; Dornan et al (2009) Blood 114(13):2721-2729; US 7521541; US 7723485; WO2009/052249). The engineered cysteine thiols may react with linker reagents or the drug-linker reagents of the present disclosure which have thiol-reactive, electrophilic groups such as maleimide or alpha-halo amides to form ADC with cysteine engineered antibodies and the PBD drug moieties. The location of the drug moiety can thus be designed, controlled, and known. The drug loading can be controlled since the engineered cysteine thiol groups typically react with thiol-reactive linker reagents or drug-linker reagents in high yield. Engineering an IgG antibody to introduce a cysteine amino acid by substitution at a single site on the heavy or light chain gives two new cysteines on the symmetrical antibody.

In some embodiments, the reactive group on the antibody may be modified to be present, for example azide. In this case, p is limited by the number of attachment sites on the antibody, i.e. the number of azide groups. For example, the antibody may have only one or two azide groups to which the drug linker may be attached.

Where more than one nucleophilic or electrophilic group of the antibody reacts with a drug-linker intermediate, or linker reagent followed by drug moiety reagent, then the resulting product is a mixture of ADC compounds with a distribution of drug moieties attached to an antibody, e.g. 1, 2, 3, etc. Liquid chromatography methods such as polymeric reverse phase (PLRP) and hydrophobic interaction (HIC) may separate compounds in the mixture by drug loading value. Preparations of ADC with a single drug loading value (p) may be isolated, however, these single loading value ADCs may still be heterogeneous mixtures because the drug moieties may be attached, via the linker, at different sites on the antibody.

Thus the antibody-drug conjugate compositions of the disclosure include mixtures of antibody-drug conjugate compounds where the antibody has one or more PBD drug moieties and where the drug moieties may be attached to the antibody at various amino acid residues.

In one embodiment, the average number of dimer pyrrolobenzodiazepine groups per antibody is in the range 1 to 8. In some embodiments the range is selected from 1 to 4, 1 to 4, 2 to 4, and 1 to 3.

In some embodiments, there are one or two dimer pyrrolobenzodiazepine groups per antibody.

Where L-D has two linking groups, these are preferably to the same antibody. In some of these embodiments, only one L-D is attached to each antibody, so the drug loading is 1.

### Preparation of Drug conjugates

The antibody drug conjugates of the present disclosure may be prepared by conjugating the the drug-linker, such as the following drug linker (of formula (II) - as previously defined herein) to the antibody:

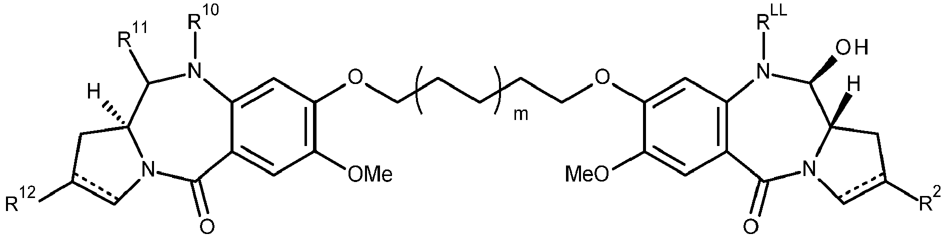

As described above, a number of conjugation techniques are know in the art, such as (i) conjugation to an endogenous or engineered cysteine residue via maleimide, as for example described in US9,889,207 or Flynn et al., 2016. Mol Cancer Ther 15: 2709 - as would be applicable to compounds C3 and C4 below; and (ii) using GlyClick or GlycoConnect to attached via chemoenzymatically-trimmed N-linked glycosylation site, e.g. at Asn-297 or its equivalent, as described in WO2018/146188 which describes the use of EndoS to trim glycan isoforms to core GlcNAc, followed by enzymatic transfer to the core GlcNAc of a N-acetylgalactose residue harboring an azide group for conjugation to the drug linker, typically using Galactose Transferase (GalT) or Galactose-N-acetyl Transferase (GalNAcT) enzyme. If a GalT enzyme is used, preferably the enzyme incorporates the Y289L and/or the C342T. Finally, the drug-linker is reacted with the azide group using copper-free click chemistry, such as the method described in van Geel, R., et al., Bioconjugate Chemistry, 2015, 26, 2233-2242. This method would be applicable to compounds C1 and C2 below.

The drug linker may be synthesised as described in, for example, Tibergien et al., 2016, ACS Med. Chem. Lett. 7: 983-987, WO2014/057074, WO2018/069490 and WO2018/146188.

In particular, the following table provides references for each of the drug-linkers of particular interest.

| | | |
|---|---|---|
| C1 | WO2018/069490 | 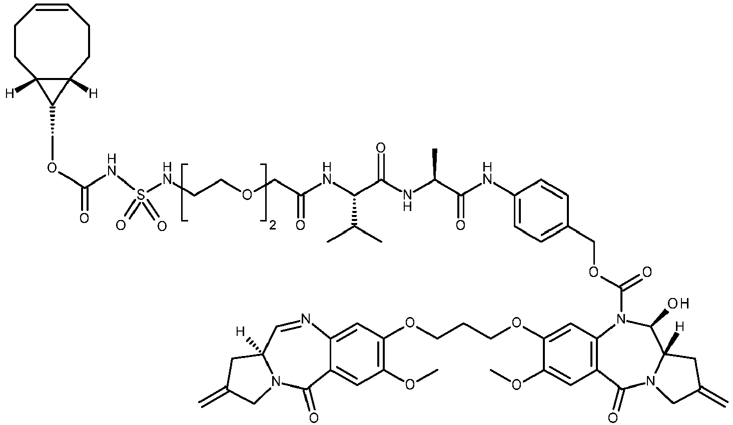 |
| | WO2018/146189 | |
| C2 | WO2018/146189 | 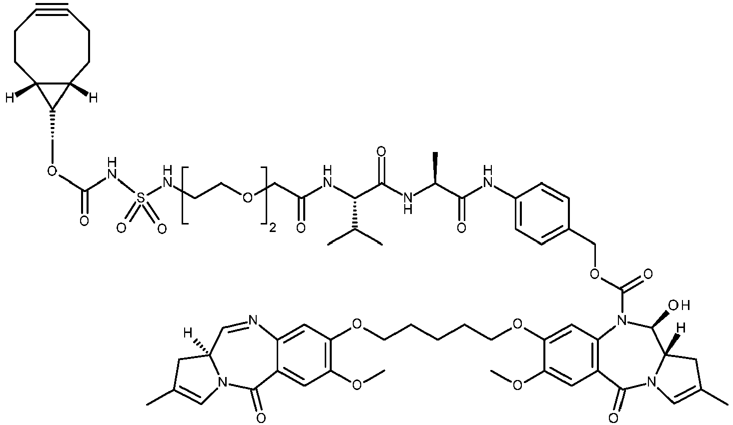 |
| C3 | WO2014/057074 | 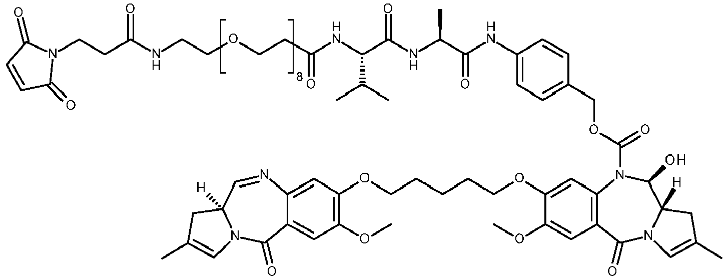 |
| | Compound 24 | |
| C4 | WO2017/137553 | 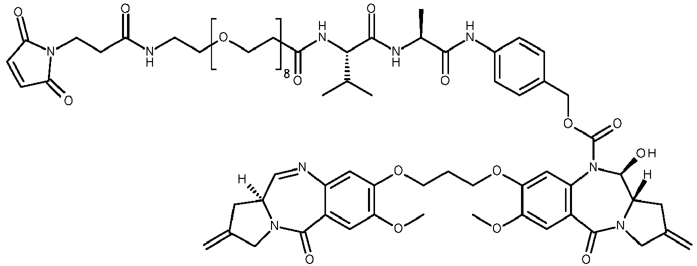 |
| | Compound 23 | |

### Cavitation Initiators

The nanoscale cavitation initiators described herein are nanoparticles having a size of from 1 nm to 1000 nm. The nanoparticles typically have a size of at least 60 nm or at least 100 nm, preferably at least 300nm and up to 1000 nm, e.g. up to 600 nm. For example, the nanoparticle may have a size of from 60 to 1000 nm or from 300 to 600 nm, preferably from 400 to 600nm or from 400 to 500 nm.

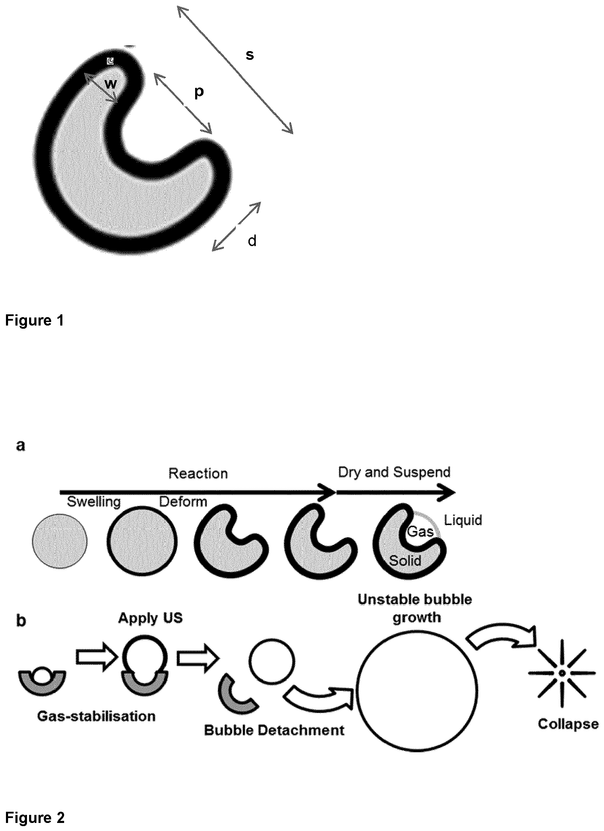

The size of the nanoparticle is typically the mean diameter of the nanoparticle (s in Figure 1). The diameter is typically determined by electron microscopy. Where a plurality of nanoparticles are present in a composition, the cumulant average is typically taken as the size of the nanoparticles. This can be determined by dynamic light scattering.

The nanoparticles comprise a single nanocup and each nanocup comprises a single gas pocket in its cavity. The use of nanoparticles having a single cup or cavity, and a single gas pocket stabilised therein, provides a more predictable and reliable response to applied ultrasound. By control of the size of the nanocup, and the size of the stabilised gas bubble, the particle can be tuned to respond to defined ultrasound conditions. Shallow or small indentations or imperfections on the surface of a nanoparticle are not considered to be cavities, in the context of the present invention. Such small indentations or imperfections typically cannot stabilise a gas pocket in their cavity. Thus, the opening of the cavity is at least 50nm in size. Typically, any indentation having a depth of less than about 5nm, typically less than about 10nm or less than about 20nm is not considered a cavity in the context of the present invention. Therefore, a nanoparticle having a single cavity may comprise one or more such indentations having an opening size of less than 50nm and/or having a depth of less than about 5nm, typically less than about 10nm or less than about 20nm, on its surface.

The nanocups are typically substantially part-spherical. The term "part-spherical" describes a shape that forms part of a sphere. The term "part-spherical" includes shapes such as a spherical cap, a hemisphere, and a sphere having its top truncated above its equator (i.e. the part of a sphere complementary to a spherical cap). Substantially part-spherical includes shapes such as those forming parts of a spheroid, and parts of a sphere or spheroid having irregularities such as depressions and protrusions.

The wall thickness of the cup refers to the shortest distance from a point on the surface of the cup facing the cavity, through the cup, to a point on the opposite surface of the cup (w in Figure 1). The depth of the cup refers to the maximum distance from the level of the rim of the cup to the base of the cup (d in Figure 1). The wall thickness and the depth of a cup can be determined by transmission electron microscopy. The wall thickness (w) of the cup is typically 10 to 100 nm, e.g. 30 to 70 nm or about 50 nm. The wall thickness of the cup can be selected and accurately controlled based on the methods disclosed herein for producing the nanoparticles.

In some embodiments the wall thickness may vary within a cup, or from cup to cup in the composition, in which case an average can be determined. In some embodiments the thickness may be uniform, or substantially uniform, within a cup or from cup to cup in the composition. The wall thickness of the cup is typically substantially uniform, both within a single nanoparticle and from one nanoparticle of the compositions of the present invention to another. The wall thickness is typically a fixed proportion of the overall cup size.

As used herein the term "substantially uniform thickness" relates to a shape with a maximum thickness that is no more than 25% greater than its minimum thickness, e.g., no more than 20% greater, no more than 15% greater, no more than 10% greater or no more than 5% greater.

The desired size and shape of the cavity containing the gas pocket is determined by the ultrasound frequency to be used, typically in the range 0.5 MHz to 5.0 MHz, in order to minimize the rarefactional pressure amplitude required to initiate inertial cavitation. Ultrasound pressure amplitudes less than 5 MPa are preferred, e.g. 4Mpa or less, or 3MPa or less. Ultrasound pressure amplitudes as low as 0.5 MPa can yield extensive inertial cavitation at frequencies as high as 2 MHz in the presence of the nanocups.

The opening of the cavity of the nanocup is the maximum dimension of the cavity measured at the rim of the cup (p in Figure 1), typically the part of the cup that forms a boundary between the encapsulated part of the gas pocket and the un-encapsulated part of the gas pocket. In the case of a substantially part-spherical cavity, the opening will be substantially circular and the size is measured as the diameter of the opening. The size of the opening of a cavity can be determined by transmission electron microscopy.

At least part of the cavity, optionally the entire cavity, is typically substantially part-spherical, e.g. substantially hemispherical or hemispherical. The cavity typically has an opening size (p), for a substantially part-spherical cavity a diameter at the opening, of from 50 to 900 nm, e.g. at least at least 100 nm and no more than 700 nm, or no more than 600 nm. The cavity may, for example, be from 50 to 700 nm or 100 to 600 nm. A skilled person will be able to select an appropriate cavity shape and size to complement desired ultrasound parameters, and produce nanoparticles having the desired cavity shapes and sizes based on the methods disclosed herein for producing the nanoparticles.

The ratio of the size of the opening to the size of the nanoparticle (ratio p:s in Figure 1) is typically from 1:3 to 5:6, for example from 1:2 to 2:3.

The cavity typically has a depth of more than 30nm, preferably more than 50nm. For example, the cavity typically has a depth of from 60 to 500nm, for example from 80 to 400nm.

The gas pocket is partially encapsulated by the nanocup. Part of the gas pocket is typically exposed to the liquid medium in which the particles are provided. This enables the expansion and collapse of the gas pocket which leads to inertial cavitation. The gas pocket is typically a bubble having a diameter of at least 50nm, preferably at least 100 or 150nm. The maximum diameter of the bubble may be no more than 300nm. In one embodiment the gas bubble is therefore from 150nm to 250nm.

The gas present in the gas pocket is not particularly limited but is typically air, nitrogen, oxygen, a perfluorocarbon such as perfluoropropane, or a mixture thereof. In one embodiment the gas pocket is an air bubble.

The gas pocket may be the same size and shape as the cavity. For example, if the cavity is a hemisphere with a diameter d at its opening, then the gas pocket may be a hemisphere of diameter d. Alternatively, the gas pocket may be larger than the cavity, i.e. it may protrude from the rim of the cup. It may protrude from the rim of the cup a distance that is 10% or more of the depth of the cup, e.g. 20% or more, 50% or more 80% or more or 100% or more. The depth of the cup and the degree to which the gas pocket may protrude can be determined using transmission electron microscopy. In some embodiments the cavity is part-spherical or substantially part-spherical with a curvature of radius r, and the gas pocket is a sphere with radius r. In an example of this embodiment the cavity is a hemisphere with a diameter d at its opening, and the gas pocket is a sphere of radius ½ *d*.

The cup is typically formed of a polymeric material, but could also be formed from any solid material that can be formed into a nanocup. Thus, the cup typically comprises a polymer, preferably it consists essentially of a polymer.

For example, the cup may be formed of a polymer capable of being formed in an emulsion polymerisation procedure. Suitable polymers include poly(methyl methacrylate) (PMMA), poly(2-hydroxyethyl methacrylate) (pHEMA), copolymers such as poly(lactic-co-glycolic) acid (PLGA), polystyrene and divinylbenzene, and methyl methacrylate/2-hydroxyethyl methacrylate copolymer, and pH or temperature responsive polymers such as poly(N-isopropylacrylamide) (PNIPAM). Other suitable polymers include, but are not limited to, polymers classed as polyanhydrides.

In some embodiments the cup is formed of a cross linked polymer. Cross linking of the polymer can be achieved by introducing a cross linking agent into the monomer mixture prior to or during polymerisation. Suitable cross linking agents include divinylbenzene (DVB) or vinylsilane. Cross linking can also be present in the polymer in the form of regions of disulphide bonds. Regions of disulfide bonds can be introduced using thiol chemistry. A cross linked polymer may be used to provide rigidity to the nanoparticle. Rigidity may be desired for example to reduce or prevent shattering of the cup during cavitation. Degree of cross linking may be determined by various spectrographic methods, such as Raman spectroscopy or nuclear magnetic resonance spectroscopy, or polymer swelling techniques.

A polymeric cup may comprise a polymer formed from two or more, for example two or three, different monomer units. The polymer may be produced by copolymerising a mixture of the monomers, and/or by providing one or more pre-polymers which are linked by co-polymerisation, or via cross-linking agents.

The cup is typically biocompatible, i.e. capable of performing its desired function with respect to a medical therapy or method of diagnosis without eliciting any therapeutically unacceptable local or systemic effects in the recipient or beneficiary of that therapy or diagnosis.

Pharmaceutical compositions comprising the nanoparticles described herein comprise, in addition to the nanoparticles, a pharmaceutically acceptable carrier or diluent, which is a liquid medium. The nanoparticles are typically provided as a suspension in the liquid medium. This facilitates delivery of the nanoparticles, which is typically by parenteral administration. Nanoparticles may optionally be stored in dried form and resuspended prior to use. A pharmaceutical composition typically comprises up to 85%, e.g. up to 50%, by weight of nanoparticles. Pharmaceutical compositions should typically be non-toxic, sterile and pyrogen-free.

Suitable pharmaceutically acceptable carriers and diluents are those suitable for parenteral administration and include aqueous solutions and sterile water. Glucose, saccharose, mannitol, glycerine, sorbitol or combinations thereof may be present in the aqueous solution. Dilute glucose solutions, e.g. 0.5-10%, preferably 1-8%, e.g. about 5% glucose solution may be used. If desired, the pharmaceutical composition may comprise one or more further excipients, carriers, buffers, stabilisers, antioxidants or other materials, as required. The pharmaceutically acceptable carriers, diluents and other excipients, buffers, stabilisers, antioxidants or other materials should be non-toxic and should not interfere with the efficacy of the nanoparticles.

Pharmaceutical compositions comprising a plurality of nanoparticles typically comprise nanoparticles which are substantially uniform, providing greater accuracy and control of acoustic properties of the particles. Typically, the particle size distribution is narrow, with a polydispersity index of no more than 0.2. The average particle size of a composition comprising a plurality of nanoparticles is defined herein as the modal average (i.e. the particle size displayed by the maximum number of particles). In a preferred aspect of the invention, at least 50% of the nanoparticles in the composition have a particle size within 50nm of the average. More preferably at least 60%, 70% or 80% of the nanoparticles have a particle size within 50nm of the average. Yet more preferably, at least 50% of the nanoparticles in the composition have a particle size within 20nm of the average. More preferably, at least 60%, 70% or 80% of the nanoparticles have a particle size within 20nm of the average. Dynamic light scattering may be used to determine the variation in particle size within a composition.

The average cavity size (the size of the opening of the cavity, p) of a composition comprising a plurality of nanoparticles is defined herein as the modal average (i.e. the cavity size displayed by the maximum number of particles). In a preferred aspect of the invention, at least 50% of the nanoparticles in the composition have a cavity size within 50nm of the average. More preferably at least 60%, 70% or 80% of the nanoparticles have a cavity size within 50nm of the average. Yet more preferably, at least 50% of the nanoparticles in the composition have a cavity size within 20nm of the average. More preferably, at least 60%, 70% or 80% of the nanoparticles have a cavity size within 20nm of the average. Dynamic light scattering may be used to determine the variation in cavity opening size within a composition.

The concentration of nanoparticles in the composition can be determined by the skilled person depending on the subject and the ultrasound parameters which are to be used. Typical compositions contain at least 0.01 mg/mL and up to 500 mg/mL. Preferably, the concentration of nanoparticles in the composition is in the range of 100 to 500 mg/mL, e.g. from 100 to 300 mg/mL.

### Process for producing nanocups

The nanoparticles can be produced by forming a cup by a seeded emulsion polymerisation technique, and drying the cup in the presence of a gas to introduce the gas pocket.

The size and shape of the cavity can be selected and accurately controlled by selecting the seed particles to be used. Thus, the seeded emulsion polymerisation technique typically uses a seed particle having a size and shape complementary with the desired size and shape of the cavity. For example, a spherical seed particle can be used to produce a cup having a part-spherical cavity. Using larger seed particles can form a larger cavity, and similarly, using smaller seed particles can produce a smaller cavity. Seed particles used will typically have a size of 50 to 900 nm, e.g. at least 50 or at least 100nm and up to 600nm, up to 400nm or up to 300nm.

Emulsion polymerisation is typically carried out by mixing seed particles, monomers, and optionally a cross linking agent in a suitable medium such as water, mixing to produce turbulent conditions and adding a suitable initiator such as potassium persulfate. The emulsion polymerisation reaction is allowed to continue at a suitable temperature for a suitable period of time. A skilled person will be able to select appropriate temperature and time parameters for the particular reaction mixture used, but the polymerisation typically takes place over 4-6 hours at a temperature of 70 to 90°C, e.g. for about 5 hours at about 80°C.

The polymerisation is typically a free radical polymerisation process. The polymers coated onto the seed particle may be solid or liquid polymers that co-polymerise with the seed particle.

Where the cup is formed from two or more monomer units, the different monomers may be provided to the emulsion polymerisation at the beginning of polymerisation, or alternatively one or more monomers may be provided part way through the polymerisation process. Alternatively, one or more of the monomer units may be pre-polymerised and a pre-polymer provided to the polymerisation mixture, either at the beginning of polymerisation or part way through polymerisation. Typically, one or more of the monomers or pre-polymers may have a greater affinity for the seed particle. Thus, the polymerisation may provide a cup having one monomer unit predominantly formed on the inner surface of the cup (i.e. the monomer having greatest affinity for the seed particle) and a different monomer unit predominantly on the exterior surface of the cup. This may provide a cup having differing surface properties on the interior and exterior surfaces.

In the case of polymeric nanocups, the seed particle may remain bound, e.g. chemically bound, to the inner surface of the nanocup, the seed particle material thus being itself incorporated into the cup. In the case of a complete reaction of all available seed particles, there will therefore be no seed particles remaining that need to be removed. Large agglomerates and small byproducts are removed through centrifugation and filtration. The final shape, size and successful formation of the nanocups can be verified by transmission electron microscopy (TEM).

The wall thickness of the cup can be controlled by selecting the amount of the monomer mixture relative to the amount of seed particles present in the reaction mixture. Raising the amount of monomer relative to the number of seed particles will increase the wall thickness of the resulting cups, and reducing the amount of monomer relative to the number of seed particles will reduce the wall thickness of the resulting cups. Additionally, the wall thickness of the cup can be adjusted by modulating the duration the polymerisation takes place, i.e., longer reaction times result in thicker cups provided that there is enough reactant.

After emulsion polymerisation has been carried out, the product can be washed, e.g., by centrifuging, in order to remove any excess polymer. After washing the product is typically suspended in a suitable medium such as water before drying.

The product can be dried by any suitable technique such as freeze-drying or air-drying. If freeze-dried a cryoprotectant is not typically used. Air-drying is preferred and may be carried out at elevated temperatures, for example, or in a desiccator. Air-drying may be carried out over a period of 12-36 hours, e.g., 24 hours. In one embodiment, air drying is carried out at a temperature of 40 to 50°C for 6 to 18 hours, e.g., about 12 hours and then in a desiccator for 32 hours at 18-24°C.

If dried in air the gas pocket formed in the cavity is an air bubble. Alternative gasses can be used for the gas pocket by carrying out the drying step in the desired gas. Alternatively, drying can be carried out in air, and the resulting nanoparticles having an air gas pocket can be subjected to a vacuum to remove the air bubble, and contacted with the desired gas to introduce a new gas pocket of the desired gas. Drying the nanocups in a gaseous atmosphere provides the required gas pocket within the cavity of the cup. Prior to drying, no gas pocket is present.

The dried nanocups may be resuspended in a liquid medium such as an aqueous solution or other suitable pharmaceutically acceptable carrier or diluent as described herein. Typically, the solution is then mixed to maximise particle dispersion. Dried nanocups retain their gas pocket on resuspension. Thus, nanocups which have been dried in a gaseous atmosphere and resuspended differ from those formed directly in solution, in that a gas pocket is present in the cavity, due to the drying step.

Following resuspension of the nanocups, the solution is preferably filtered to remove agglomerated material. Syringe filtration, for example at 1-5µm, typically 1.5-3µm, may be used to remove fine particles. This limits the particles present to those having a nanoparticle size of less than the filtration limit. A combination of coarse filtration followed by syringe filtration may be used.

Figure 2 schematically depicts the process of producing the nanocups according to the invention. Fig 2a shows individual template particles which were coated with a co-polymer, which induced swelling and deformation of the spherical particle. The resulting nanocup was dried and suspended to entrap gas in the cavity. Fig 2b gives a diagrammatic representation of the proposed mechanism for inertial cavitation for nanoparticle systems. Initially the bubble is stabilized within the cavity. With enough negative pressure, the bubble can extend onto the mouth of the cavity, and eventually detach from the particle. For inertial cavitation to occur, the detached bubble must grow unstably before finally collapsing under the inertia of the surrounding medium.

### Treated disorders

The therapies described herein include those with utility for anticancer activity. In particular, in certain aspects the therapies include an antibody conjugated, i.e. covalently attached by a linker, to a PBD drug moiety, i.e. toxin. When the drug is not conjugated to an antibody, the PBD drug has a cytotoxic effect. The biological activity of the PBD drug moiety is thus modulated by conjugation to an antibody. The antibody-drug conjugates (ADC) of the disclosure selectively deliver an effective dose of a cytotoxic agent to tumor tissue whereby greater selectivity, i.e. a lower efficacious dose, may be achieved.

Administration of the ADC in combination with nanoparticles and ultrasound enables enhanced and targeted delivery of the ADC to the tumour site, enabling a yet lower dose to be used. Following administration of both ADC and nanoparticles, ultrasound is applied at the diseased site. This causes inertial cavitation in the treated area. Microstreaming, resulting from inertial cavitation effects, enhances transport of both nanoparticles and co-administered ADC into the tumour. The inertial cavitation may also deliver ADC a further distance into the tumour mass.

Thus, the present disclosure provides therapies for the treatment of solid tumour, comprising administering (i) a composition comprising a plurality of nanoparticles as described herein (also referred to as the nanoparticle composition), and (ii) the conjugate as described herein, and exposing the subject to ultrasound.

Also provided is a method of treating solid tumour in a subject, the method comprising administering (i) the nanoparticle composition as described herein, and (ii) the conjugate as described herein, and exposing the subject to ultrasound. A therapeutically effective amount of the conjugate is administered to the subject. The amount of the nanoparticle composition which is administered to the subject is an amount effective to induce inertial cavitation on exposure to ultrasound.

Also provided herein is the use of a conjugate as described herein in the manufacture of a medicament for use in a method of treating solid tumour in a subject, the method comprising administering (i) a nanoparticle composition as described herein, and (ii) the conjugate as described herein, and exposing the subject to ultrasound.

Also provided is the nanoparticle composition as described herein for use in a method of treating solid tumour in a subject, the method comprising administering (i) the nanoparticle composition as described herein, and (ii) a conjugate as described herein, and exposing the subject to ultrasound. Also provided herein is the use of the nanoparticle composition as described herein for the manufacture of a medicament for use in a method comprising administering (i) the nanoparticle composition as described herein, and (ii) a conjugate as described herein, and exposing the subject to ultrasound.

The therapies described herein may be used to treat a proliferative disease. The term "proliferative disease" pertains to an unwanted or uncontrolled cellular proliferation of excessive or abnormal cells which is undesired, such as, neoplastic or hyperplastic growth, whether *in vitro* or *in vivo.*

The proliferative disease may be characterised by the presence of a neoplasm comprising both AXL+ve and AXL-ve cells.

The target neoplasm or neoplastic cells may be all or part of a solid tumour, such as an advanced solid tumour.

Thus in one embodiment the neoplasm/cancer is itself essentially AXL+ve.

Examples of proliferative conditions that may be treated using the methods and conjugate compounds described herein include, but are not limited to, benign, pre-malignant, and malignant cellular proliferation, including but not limited to, neoplasms, tumours and cancers. In one embodiment, the proliferative condition is a solid tumour.

Particular proliferative conditions to be treated include lung cancer (such as non-small cell lung cancer), breast cancer (such as triple negative breast cancer), pancreatic cancer, ovarian cancer, prostate cancer, colon cancer, colorectal cancer, renal cell carcinoma, thymic carcinoma, glioblastoma and melanoma.

### Patient Selection

In certain aspects, the individuals are selected as suitable for treatment with the treatments before the treatments are administered.

As used herein, individuals who are considered suitable for treatment are those individuals who are expected to benefit from, or respond to, the treatment. Individuals may have, or be suspected of having, or be at risk of having cancer. Individuals may have received a diagnosis of cancer. Typically the individual is an animal or human subject.

In some aspects, individuals are selected on the basis of the amount or pattern of expression of a first target protein. In some aspects, the selection is based on expression of a first target protein at the cell surface.

In some cases, individuals are selected on the basis they have, or are suspected of having, are at risk of having cancer, or have received a diagnosis of a proliferative disease characterised by the presence of a neoplasm comprising cells having a high level of surface expression of AXL. The neoplasm may be composed of cells having a high level of surface expression of AXL. In some cases, high levels of surface expression means that mean number of anti-AXL antibodies bound per neoplastic cell is greater than 70000, such as greater than 80000, greater than 90000, greater than 100000, greater than 110000, greater than 120000, greater than 130000, greater than 140000, or greater than 150000.

In some cases, individuals are selected on the basis they have, or are suspected of having, are at risk of having cancer, or have received a diagnosis of a proliferative disease characterised by the presence of a neoplasm comprising cells having a low level of surface expression of AXL. The neoplasm may be composed of cells having a low level of surface expression of AXL. In some cases, low levels of surface expression means that mean number of anti-AXL antibodies bound per neoplastic cell is less than 20000, such as less than 80000, less than 70000, less than 60000, less than 50000, less than 40000, less than 30000, less than 20000, less than 10000, or less than 5000.

In some aspects, individuals are selected on the basis they have a neoplasm comprising both AXL+ve and AXL-ve cells. The neoplasm may be composed of AXL-ve neoplastic cells, optionally wherein the AXL-ve neoplastic cells are associated with AXL+ve neoplastic or non-neoplastic cells. The neoplasm or neoplastic cells may be all or part of a solid tumour. The solid tumour may be partially or wholly AXL-ve.

In some cases, expression of AXL in a particular tissue of interest is determined. For example, in a sample of tumor tissue. In some cases, systemic expression of the target is determined. For example, in a sample of circulating fluid such as blood, plasma, serum or lymph.

In some aspects, the individual is selected as suitable for treatment due to the presence or absence of AXL expression in a sample. In those cases, individuals without AXL expression may be considered not suitable for treatment.

In other aspects, the level of AXL expression is used to select a individual as suitable for treatment. Where the level of expression of AXL is above a threshold level, the individual is determined to be suitable for treatment.

In some aspects, the presence of AXL in cells in the sample indicates that the individual is suitable for treatment with an ADC as disclosed herein. In other aspects, the amount of AXL expression must be above a threshold level to indicate that the individual is suitable for treatment. In some aspects, the observation that AXL localisation is altered in the sample as compared to a control indicates that the individual is suitable for treatment.

In some aspects, a patient is determined to be suitable for treatment if at least 5%, 10%, 15%, 20%, 25%, 30%, 35%, 40%, 45%, 50%, 55%, 60%, 65%, 70%, 75%, 80%, 85%, 90% or more of all cells in the sample express a first target protein. In some aspects disclosed herein, a patient is determined to be suitable for treatment if at least at least 10% of the cells in the sample express a first target protein.

In some aspects, a patient is determined to be suitable for treatment if at least 5%, 10%, 15%, 20%, 25%, 30%, 35%, 40%, 45%, 50%, 55%, 60%, 65%, 70%, 75%, 80%, 85%, 90% or more of all cells in the sample express a second target protein. In some aspects disclosed herein, a patient is determined to be suitable for treatment if at least at least 10% of the cells in the sample express a second target protein.

In some aspects the sample is taken from a bodily fluid, more preferably one that circulates through the body. Accordingly, the sample may be a blood sample or lymph sample. In some cases, the sample is a urine sample or a saliva sample.

In some cases, the sample is a blood sample or blood-derived sample. The blood derived sample may be a selected fraction of a individual's blood, e.g. a selected cell-containing fraction or a plasma or serum fraction.

A selected cell-containing fraction may contain cell types of interest which may include white blood cells (WBC), particularly peripheral blood mononuclear cells (PBC) and/or granulocytes, and/or red blood cells (RBC). Accordingly, methods according to the present disclosure may involve detection of a first target polypeptide or nucleic acid in the blood, in white blood cells, peripheral blood mononuclear cells, granulocytes and/or red blood cells.

In another aspect the sample is a biopsy of solid tissue.

The sample may be fresh or archival. For example, archival tissue may be from the first diagnosis of an individual, or a biopsy at a relapse. In certain aspects, the sample is a fresh biopsy.

The terms "subject", "patient" and "individual" are used interchangeably herein.

In some aspects disclosed herein, an individual has, or is suspected as having, or has been identified as being at risk of, a proliferative disease such as cancer. In some aspects disclosed herein, the individual has already received a diagnosis of such a disease. A list of relevant diseases is provided above. Glioblastoma (e.g. Glioblastoma multiforme), ovarian cancer, breast cancer, colon cancer, pancreatic cancer, thyroid cancer and melanoma are conditions of particular interest.

In some cases, the individual has received a diagnosis of a proliferative disease such as cancer, such as one of the disorders listed above. Glioblastoma (e.g. Glioblastoma multiforme), ovarian cancer, breast cancer, colon cancer, pancreatic cancer, thyroid cancer and melanoma are conditions of particular interest.

In some cases, the individual has received a diagnosis of a solid cancer containing AXL+ expressing cells.

The individual may be undergoing, or have undergone, a therapeutic treatment for that cancer. The subject may, or may not, have previously received an anti-AXL ADC. In some cases the cancer is Glioblastoma (e.g. Glioblastoma multiforme), ovarian cancer, breast cancer, colon cancer, pancreatic cancer, thyroid cancer or melanoma.

### Methods of Treatment

The term "treatment," as used herein in the context of treating a condition, pertains generally to treatment and therapy, whether of a human or an animal (e.g., in veterinary applications), in which some desired therapeutic effect is achieved, for example, the inhibition of the progress of the condition, and includes a reduction in the rate of progress, a halt in the rate of progress, regression of the condition, amelioration of the condition, and cure of the condition. Treatment as a prophylactic measure (i.e., prophylaxis, prevention) is also included.

The term "therapeutically-effective amount" or "effective amount" as used herein, pertains to that amount of an active compound, or a material, composition or dosage from comprising an active compound, which is effective for producing some desired therapeutic effect, commensurate with a reasonable benefit/risk ratio, when administered in accordance with a desired treatment regimen.

Similarly, the term "prophylactically-effective amount," as used herein, pertains to that amount of an active compound, or a material, composition or dosage from comprising an active compound, which is effective for producing some desired prophylactic effect, commensurate with a reasonable benefit/risk ratio, when administered in accordance with a desired treatment regimen.

Disclosed herein are methods of therapy. Also provided is a method of treatment, comprising administering to a subject in need of treatment a therapeutically-effective amount of an ADC and a composition comprising nanoparticles as described herein. The term "therapeutically effective amount" is an amount sufficient to show benefit to a subject. Such benefit may be at least amelioration of at least one symptom. The actual amount administered, and rate and time-course of administration, will depend on the nature and severity of what is being treated. Prescription of treatment, e.g. decisions on dosage, is within the responsibility of general practitioners and other medical doctors. The subject may have been tested to determine their eligibility to receive the treatment according to the methods disclosed herein. The method of treatment may comprise a step of determining whether a subject is eligible for treatment, using a method disclosed herein. The nanoparticle composition is administered in an amount effective to induce inertial cavitation. Inertial cavitation may be monitored and detected using passive acoustic mapping (WO2010/052494).

The treatment may involve administration of the ADC (and the nanoparticle composition) alone or in combination with other treatments, either simultaneously or sequentially dependent upon the condition to be treated. Examples of treatments and therapies include, but are not limited to, chemotherapy (the administration of active agents, including, e.g. drugs, such as chemotherapeutics); surgery; and radiation therapy.

A "chemotherapeutic agent" is a chemical compound useful in the treatment of cancer, regardless of mechanism of action. Classes of chemotherapeutic agents include, but are not limited to: alkylating agents, antimetabolites, spindle poison plant alkaloids, cytotoxic/antitumor antibiotics, topoisomerase inhibitors, antibodies, photosensitizers, and kinase inhibitors. Chemotherapeutic agents include compounds used in "targeted therapy" and conventional chemotherapy.

Also included in the definition of "chemotherapeutic agent" are: (i) anti-hormonal agents that act to regulate or inhibit hormone action on tumors such as anti-estrogens and selective estrogen receptor modulators (SERMs); (ii) aromatase inhibitors that inhibit the enzyme aromatase, which regulates estrogen production in the adrenal glands; (iii) anti-androgens; (iv) protein kinase inhibitors such as MEK inhibitors; (v) lipid kinase inhibitors; (vi) anti-angiogenic agents).

Also included in the definition of "chemotherapeutic agent" are therapeutic antibodies.

Compositions comprising the conjugate compound are preferably pharmaceutical compositions. Pharmaceutical compositions according to the present disclosure, and for use in accordance with the present disclosure, may comprise, in addition to the active ingredient, e.g. a conjugate compound, a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which will typically be by injection, e.g. cutaneous, subcutaneous, or intravenous.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

The nanoparticle composition may be administered by injection, for example by intravenous, cutaneous or subcutaneous administration, typically it is administered by intravenous administration. The nanoparticle composition and the conjugate are typically administered separately, and may be administered concurrently, sequentially or there may be a period of time between administration of the two agents. The nanoparticle composition may be administered before the conjugate. Alternatively, the conjugate may be administered before the nanoparticle composition.

Following administration of the conjugate and the nanoparticle composition, the subject is exposed to ultrasound at a pressure and frequency suitable for clinical use. Ultrasound is applied at the diseased site. Typically, low-intensity focussed ultrasound is used. Low intensity ultrasound typically has an intensity similar to or lower than that used in routine clinical practice for diagnostic applications. Ultrasound may be pulsed or continuous.

Ultrasound pressure amplitudes less than 5 MPa are preferred, e.g. 4Mpa or less, or 3MPa or less. Thus, typical peak negative ultrasound pressure is in the range of from 0.5MPa to 5MPa, e.g. from 1 to 4MPa, e.g. from 1.5 to 3 MPa. The ultrasound frequency (fc) may be up to 5MHz, e.g. up to 2MHz, e.g. up to 1MHz or about 0.5 MHz. Typicaly frequencies used (fc) are in the range of 0.1 to 5 MHz, e.g. from 0.1 to 1MHz. The pulse repetition frequency may be up to 20 kHz, for example up to 1kHz or up to 1Hz. Ultrasound exposure may continue for up to 120 minutes, e.g. up to 60 minutes, or up to 30 minutes. Typically the ultrasound exposure is carried out at a time when there will be effective circulation of the conjugate, and the nanoparticle composition, in the subject's bloodstream. Preferably, the ultrasound administration is carried out during the period of peak circulation of the conjugate.

Cavitation may be monitored during exposure, for example using passive acoustic mapping (WO2010/052494). Passive acoustic mapping may be used to maintain the cavitation energy within a desired range, for example below 1nJ, or within the range 0.1 pJ to 1 nJ, for example from 0.5 pJ to 0.5 nJ. Variation of the peak negative pressure ultrasound can be used to control the cavitation energy. Passive acoustic mapping may also be used to monitor in real time the delivery of conjugate to the tumour. By monitoring cavitation in the subject, the extent and location of conjugate delivery can be determined.

### Dosage

It will be appreciated by one of skill in the art that appropriate dosages of the ADC, and compositions comprising these active elements, can vary from subject to subject. Determining the optimal dosage will generally involve the balancing of the level of therapeutic benefit against any risk or deleterious side effects. The selected dosage level will depend on a variety of factors including, but not limited to, the activity of the particular compound, the route of administration, the time of administration, the rate of excretion of the compound, the duration of the treatment, other drugs, compounds, and/or materials used in combination, the severity of the condition, and the species, sex, age, weight, condition, general health, and prior medical history of the subject. The amount of compound and route of administration will ultimately be at the discretion of the physician, veterinarian, or clinician, although generally the dosage will be selected to achieve local concentrations at the site of action which achieve the desired effect without causing substantial harmful or deleterious side-effects.

In certain aspects, the dosage of ADC is determined by the expression of AXL observed in a sample obtained from the subject. Thus, the level or localisation of expression of AXL in the sample may be indicative that a higher or lower dose of ADC is required. For example, a high expression level of AXL may indicate that a higher dose of ADC would be suitable. In some cases, a high expression level of AXL may indicate the need for administration of another agent in addition to the ADC. For example, administration of the ADC in conjunction with a chemotherapeutic agent. A high expression level of AXL may indicate a more aggressive therapy.

In certain aspects, the dosage level is determined by the expression of AXL on neoplastic cells in a sample obtained from the subject. For example, when the target neoplasm is composed of, or comprises, neoplastic cells expressing AXL.

In certain aspects, the dosage level is determined by the expression of AXL on cells associated with the target neoplasm. For example, the target neoplasm may be a solid tumour composed of, or comprising, neoplastic cells that express AXL. For example, the target neoplasm may be a solid tumour composed of, or comprising, neoplastic cells that do not express AXL. The cells expressing AXL may be neoplastic or non-neoplastic cells associated with the target neoplasm.

Administration of the conjugate can be effected in one dose, continuously or intermittently (e.g., in divided doses at appropriate intervals) throughout the course of treatment. Methods of determining the most effective means and dosage of administration of conjugate are well known to those of skill in the art and will vary with the formulation used for therapy, the purpose of the therapy, the target cell(s) being treated, and the subject being treated. Single or multiple administrations can be carried out with the dose level and pattern being selected by the treating physician, veterinarian, or clinician. Where the conjugate is administered in multiple doses throughout a course of treatment, the nanoparticle composition, and application of ultrasound, may be provided together with one dose of conjugate, or two or more doses of conjugate may be administered together with the nanoparticle composition and ultrasound.

For the ADC, the dosage amounts described above may apply to the conjugate (including the PBD moiety and the linker to the antibody) or to the effective amount of PBD compound provided, for example the amount of compound that is releasable after cleavage of the linker.

Aspects of the Invention:
1. An antibody drug conjugate for use in a method of treating a solid tumour in a subject, which method comprises administering to the subject:
   (i) a pharmaceutical composition comprising a plurality of nanoparticles, each nanoparticle comprising:
      a cup having a cavity, wherein the cavity has an opening of at least 50nm in size, and
      a gas pocket present in the cavity,
      wherein the gas pocket is partially encapsulated by the cup, wherein the nanoparticle comprises a single nanocup having a single cavity, and wherein the pharmaceutical composition further comprises a pharmaceutically acceptable carrier or diluent, which is a liquid medium; and
   (ii) the antibody drug conjugate, wherein the antibody drug conjugate comprises an anti-AXL antibody conjugated to a pyrrolobenzodiazepine dimer;
      and exposing the subject to ultrasound.
2. The antibody drug conjugate for use according to [1] wherein the antibody comprises (i) an immunoglobulin heavy chain variable region having a CDR1 region with the amino acid sequence shown in SEQ ID NO: 3, a CDR2 region with the amino acid sequence shown in SEQ ID NO: 4, and a CDR3 region with the amino acid sequence shown in SEQ ID NO: 5; and (ii) an immunoglobulin light chain variable region having a CDR1 region with the amino acid sequence shown in SEQ ID NO: 6, a CDR2 region with the amino acid sequence shown in SEQ ID NO: 7, and a CDR3 region with the amino acid sequence shown in SEQ ID NO: 8.
3. The antibody drug conjugate for use according to [2] wherein the antibody comprises an immunoglobulin heavy chain variable region having the amino acid sequence shown in SEQ ID NO: 1.
4. The antibody drug conjugate for use according to [2] or [3] wherein the antibody comprises an immunoglobulin light chain variable region having the amino acid sequence shown in SEQ ID NO:2.
5. The antibody drug conjugate for use according to any one of [1] to [4] where the antibody drug conjugate is of formula (I):

   Ab - L-D (I)

   wherein:
   Ab is an antibody that binds to AXL; and
   L-D is a drug linker of formula (II): 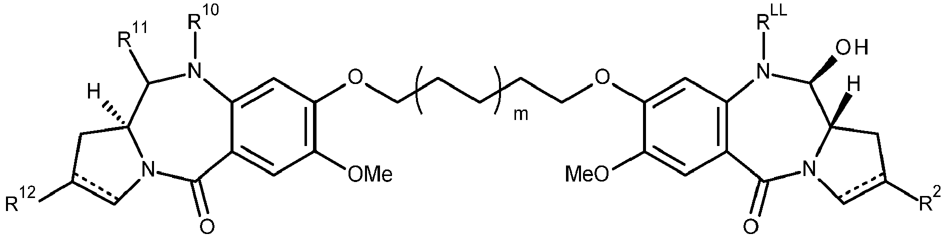 wherein:
      (a) R^{LL} is a linker for connection to Ab;
      (b) (i) R¹⁰ and R¹¹ together form a double bond between the nitrogen and carbon atoms to which they are attached; or (ii) R¹⁰ is R^{LLA} which is a linker for connection to Ab, and R¹¹ is OH; or (iii) R¹⁰ is a capping group R^{C}, and R¹¹ is OH;
      (c) m is 0 or 1; and
      (d) when there is a double bond between C2 and C3, R² is methyl;

      when there is a single bond between C2 and C3, R² is either H or 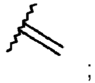
      when there is a double bond between C2' and C3', R¹² is methyl;
      when there is a single bond between C2' and C3', R¹² is H or 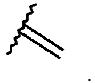
6. The antibody drug conjugate for use according to [5], wherein R^{LL} is of formula (IIa): 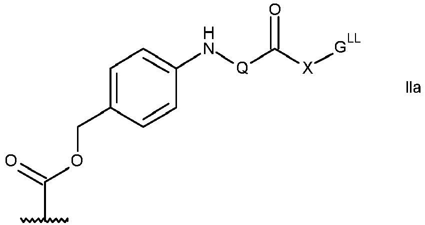 wherein:
   Q is: 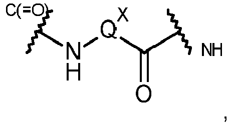 where Q^{x} is such that Q is an amino-acid residue, a dipeptide residue, a tripeptide residue or a tetrapeptide residue;
   X is: 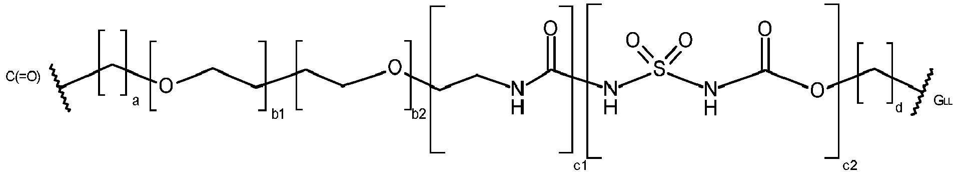 where a = 0 to 5, b1 = 0 to 16, b2 = 0 to 16, c1 = 0 or 1, c2 = 0 or 1, d = 0 to 5, wherein at least b1 or b2 = 0 and at least c1 or c2 = 0; and
   G^{LL} is a linker group connected to the antibody.
7. The antibody drug conjugate for use according to any one of [1] to [6] wherein the linker is a cleavable linker, such as a linker comprising a cathepsin cleavable sequence e.g. Val-Ala or Val-Cit.
8. The antibody drug conjugate for use according to any one of [1] to [7] where the cytotoxin is conjugated to the antibody at an endogenous and/or engineered N-linked glycosylation site.
9. The antibody drug conjugate for use according to any one of [5] to [8] where L-D is compound B2.
10. The antibody drug conjugate for use according to any one of [1] to [9], wherein at least a part of the cavity of each nanoparticle is substantially part-spherical, preferably wherein the cavity of each nanoparticle is substantially part-spherical.
11. The antibody drug conjugate for use according to any one of [1] to [10], wherein the gas pocket is a bubble having a diameter of at least 50nm, preferably from 150 to 250nm.
12. The antibody drug conjugate for use according to any one of [1] to [11], wherein the gas pocket is an air bubble.
13. The antibody drug conjugate for use according to any one of [1] to [12], wherein the cup of each nanoparticle is substantially part-spherical.
14. The antibody drug conjugate for use according to any one of [1] to [13], wherein each cup has a substantially uniform wall thickness.
15. The antibody drug conjugate for use according to any one of [1] to [14], wherein each nanoparticle is from 300 to 600nm in size.
16. The antibody drug conjugate for use according to any one of [1] to [15], wherein the nanoparticles are polymeric nanoparticles, preferably the nanoparticles comprise cross-linked polymer.
17. The antibody drug conjugate for use according to any one of [1] to [16], wherein the ultrasound has a pressure amplitude of from 1MPa to 4MPa.
18. A method of treating a solid tumour in a subject, which method comprises administering to the subject:
   (i) a pharmaceutical composition comprising a plurality of nanoparticles, each nanoparticle comprising:
      a cup having a cavity, wherein the cavity has an opening of at least 50nm in size, and
      a gas pocket present in the cavity,
      wherein the gas pocket is partially encapsulated by the cup, wherein the nanoparticle comprises a single nanocup having a single cavity, and wherein the pharmaceutical composition further comprises a pharmaceutically acceptable carrier or diluent, which is a liquid medium; and (ii) an antibody drug conjugate comprising an anti-AXL antibody conjugated to a pyrrolobenzodiazepine dimer;
      and exposing the subject to ultrasound.

### Sequence listing part of the description

SEQ ID NO.1 [1H12 VH, CDR underline] 
QVQLVESGGG VVQPGRSLRL SCAASGFTFS SYGMSWVRQA PGKGLEWVAT ISSGGSYTYY 60
PDSVKGRFTI SRDNSKNTLY LQMNSLRAED TAVYYCARHP IYYTYDDTMD YWGQGTTVTV 120
SS 122
SEQ ID NO.2 [1H12 VL. CDR underline] 
EIVLTQSPGT LSLSPGERAT LSCSASSSVS SGNFHWYQQK PGLAPRLLIY RTSNLASGIP 60
ARFSGSGSGT DFTLTISSLE PEDFAVYYCQ QWSGYPWTFG GGTKLEIK 108
SEQ ID NO.3 [1H12 VH, Kabat CDR1]
   GFTFSSYGMS
SEQ ID NO.4 [1H12 VH, Kabat CDR2]
   TISSGGSYTYYPDSVKGRFTI SRDNS
SEQ ID NO.5 [1H12 VH, Kabat CDR3]
   HPIYYTYDDT MDY
SEQ ID NO.6 [1H12 VL. Kabat CDR1]
   SASSSVSSGN FH
SEQ ID NO.7 [1H12 VL. Kabat CDR2]
   RTSNLAS
SEQ ID NO.8 [1H12 VL. Kabat CDR3]
   QQWSGYPWT
SEQ ID NO.9 [1H12 Full Length Heavy Chain] 
QVQLVESGGG VVQPGRSLRL SCAASGFTFS SYGMSWVRQA PGKGLEWVAT ISSGGSYTYY 60
PDSVKGRFTI SRDNSKNTLY LQMNSLRAED TAVYYCARHP IYYTYDDTMD YWGQGTTVTV 120
SSASTKGPSV FPLAPSSKST SGGTAALGCL VKDYFPEPVT VSWNSGALTS GVHTFPAVLQ 180
SSGLYSLSSV VTVPSSSLGT QTYICNVNHK PSNTKVDKKV EPKSCDKTHT CPPCPAPELL 240
GGPSVFLFPP KPKDTLMISR TPEVTCVVVD VSHEDPEVKF NWYVDGVEVH NAKTKPREEQ 300
YNSTYRVVSV LTVLHQDWLN GKEYKCKVSN KALPAPIEKT ISKAKGQPRE PQVYTLPPSR 360
EEMTKNQVSL TCLVKGFYPS DIAVEWESNG QPENNYKTTP PVLDSDGSFF LYSKLTVDKS 420
RWQQGNVFSC SVMHEALHNH YTQKSLSLSP G 451
SEQ ID NO.10 [1H12 Full Length Light Chain] EIVLTQSPGT LSLSPGERAT LSCSASSSVS SGNFHWYQQK PGLAPRLLIY RTSNLASGIP 60 
ARFSGSGSGT DFTLTISSLE PEDFAVYYCQ QWSGYPWTFG GGTKLEIKRT VAAPSVFIFP 120
PSDEQLKSGT ASVVCLLNNF YPREAKVQWK VDNALQSGNS QESVTEQDSK DSTYSLSSTL 180
TLSKADYEKH KVYACEVTHQ GLSSPVTKSF NRGEC 215
SEQ ID NO: 11 [AXL]

MAWRCPRMGR VPLAWCLALC GWACMAPRGT QAEESPFVGN PGNITGARGL TGTLRCQLQV 60
QGEPPEVHWL RDGQILELAD STQTQVPLGE DEQDDWIVVS QLRITSLQLS DTGQYQCLVF 120
LGHQTFVSQP GYVGLEGLPY FLEEPEDRTV AANTPFNLSC QAQGPPEPVD LLWLQDAVPL 180
ATAPGHGPQR SLHVPGLNKT SSFSCEAHNA KGVTTSRTAT ITVLPQQPRN LHLVSRQPTE 240
LEVAWTPGLS GIYPLTHCTL QAVLSDDGMG IQAGEPDPPE EPLTSQASVP PHQLRLGSLH 300
PHTPYHIRVA CTSSQGPSSW THWLPVETPE GVPLGPPENI SATRNGSQAF VHWQEPRAPL 360
QGTLLGYRLA YQGQDTPEVL MDIGLRQEVT LELQGDGSVS NLTVCVAAYT AAGDGPWSLP 420
VPLEAWRPGQ AQPVHQLVKE PSTPAFSWPW WYVLLGAVVA AACVLILALF LVHRRKKETR 480
YGEVFEPTVE RGELVVRYRV RKSYSRRTTE ATLNSLGISE ELKEKLRDVM VDRHKVALGK 540
TLGEGEFGAV MEGQLNQDDS ILKVAVKTMK IAICTRSELE DFLSEAVCMK EFDHPNVMRL 600
IGVCFQGSER ESFPAPVVIL PFMKHGDLHS FLLYSRLGDQ PVYLPTQMLV KFMADIASGM 660
EYLSTKRFIH RDLAARNCML NENMSVCVAD FGLSKKIYNG DYYRQGRIAK MPVKWIAIES 720
LADRVYTSKS DVWSFGVTMW EIATRGQTPY PGVENSEIYD YLRRGNRLKQ PADCLDGLYA 780
LMSRCWELNP QDRPSFTELR EDLENTLKAL PPAQEPDEIL YVNMDEGGGY PEPPGAAGGA 840
DPPTQPDPKD SCSCLTAAEV HPAGRYVLCP STTPSPAQPA DRGSPAAPGQ EDGA 894

Some aspects and embodiments of the disclosure are described below in more detail with reference to the following examples, which are illustrative only and non-limiting.

### Examples

### Synthesis of ADC

AXL-PBD was constructed as described in WO2018/146189 (with anti-AXL antibody 1H12 and PBD SG3199 - see compound C2).

### Control Antibody

Control antibody was anti-AXL antibody 1H12.

### Synthesis of Nanoparticles

Nanoparticles were prepared using the method of Example 1 of WO 2015/075442. The nanoparticles were 480nm in size, with 200nm air bubbles stabilised in the cavity. After drying, nanoparticles were provided in 5% glucose solution at a concentration of 17.5mg/uL.

### Objective

In this study, a low single dose of ADC was tested in combination with nanoparticles plus ultrasound treatment in the tumour xenograft model SN12C (human renal cancer). The therapeutic regime was compared to the corresponding antibody without exposure to ultrasound (at both low and high dose), to understand if the combination treatment showed enhanced therapeutic effect over antibody alone at the same dose.

### Method

Cells were thawed and expanded in vitro and, on the day of tumour implantation, harvested, counted and resuspended at the concentration of 5×10⁷ cells/mL in Matrigel-PBS mixture (1:1). The prepared cell suspension was kept in bijou tubes on ice at all times. Female CB17 SCID mice (7-9 weeks of age; n = 50) were shaved a day before cell implantation (back and flanks areas). On the procedure day, mice were anaesthetized and a microchip was implanted subcutaneously in the neck/back area. While still under anesthesia, they were injected subcutaneously with SN12C cells (5×10⁶ cells in 100µL) on one flank (unilateral tumours) using a 1 mL syringe mounted with a 25G needle. Animals were monitored 2-3 times per week, involving general welfare checks, caliper measuring of tumours and mouse weighing. When tumours reached an average size of approximately 150mm³, animals were divided into 6 cohorts as shown in Table 1.

Mice were depilated 4 days before the treatment. On the day of treatment, mice were kept in a hot box (40°C) for 10-15 minutes to induce vasodilation before being anaesthetized. While under anaesthesia, all mice were placed in a water tank (37°C) and intravenously injected (via tail vein cannuation) with nanoparticles (0.875mg in 50µl of glucose 5%) and either PBS (conrol group), ADC or antibody. Subsenquently, every animal was exposed to ultrasound (US) or mock treatment (no US).

**Table 1: Experimental groups.**

| **Group** | **Drug** | **# Mice** | **US Treatment** | **Endpoint** | **Role in the experiment** |
|---|---|---|---|---|---|
| 1 | PBS Control | 6 | No | Tumour growth | No treatment ("normal" tumour growth). |
| 2 | Antibody (0.3mg/kg) | 7 | No | Tumour growth | Control antibody ("normal" tumour growth) |
| 3 | Antibody (0.3mg/kg) | 7 | Yes | Tumour growth | Control antibody + ultrasound |
| 4 | ADC (0.3 mg/kg) | 7 | No | Tumour growth | Effect of ADC and its passive accumulation |
| 5 | ADC (0.3 mg/kg) | 7 | Yes | Tumour growth | Effect of ADC + ultrasound combination on efficacy |
| 6 | ADC (1 mg/kg) | 7 | No | Tumour growth | Effect of ADC and its passive accumulation |

On infusion of nanoparticles and drug agent, mice which received US treatment were exposed to pulsed therapeutic ultrasound (fc=0.5MHz, N=8,000cycles, repetition 0.5Hz) for 10 minutes with variable peak negative ultrasound pressure between 1.5MPa to 3MPa. Cavitation was monitored by passive acoustic mapping and maintained between 0.1 to 0.5 nJ by modification of ultrasound pressure, as needed. After treatment, all mice were placed back into their cages to recover.

Mice were monitored 2 times a week and tumours measured by caliper until the average between tumour length and width passed the value of 12mm. Mice were then culled and tumours were collected and immediately frozen (-20°C).

### Results

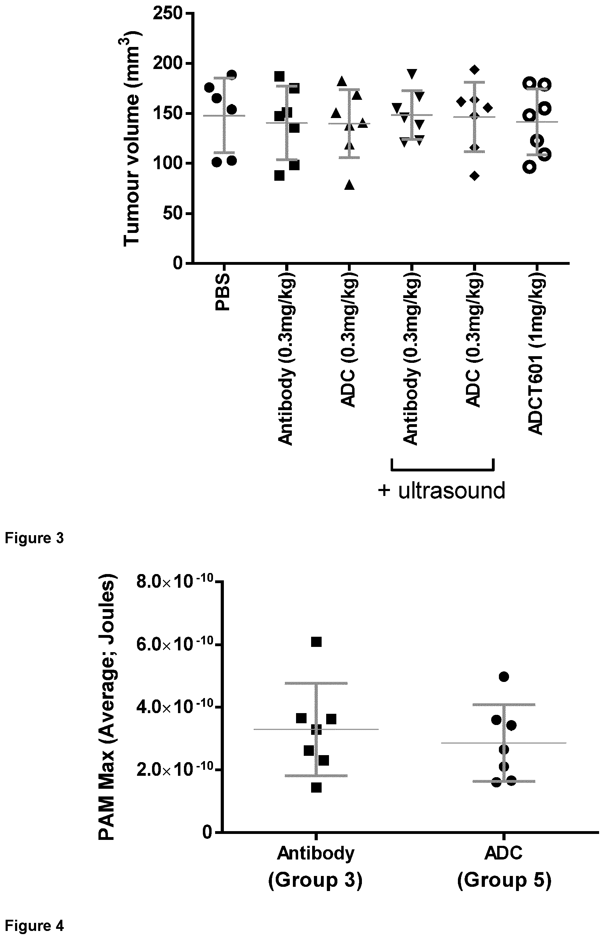

Average tumour volume the day before treatment was around 140-150nm³ for all groups as shown in Figure 3. In Figure 3, each single dot represents an individual tumour, while bars indicate group means ± standard deviation. One-way ANOVA test was used for the statistical analysis, no statistical significance was found.

Cavitation was recorded by passive acoustic mapping and is shown in Figure 4. Cavitation in US-treated groups was similar and within the set target range of 1×10⁻¹⁰ - 5×10⁻¹⁰ Joules for both group 3 and 5 (antibody and ADC respectively). In Figure 4, each single dot represents an individual tumour, while bars indicate group means ± standard deviation. Unpaired t-test was used for the statistical analysis, no statistical significance was found.

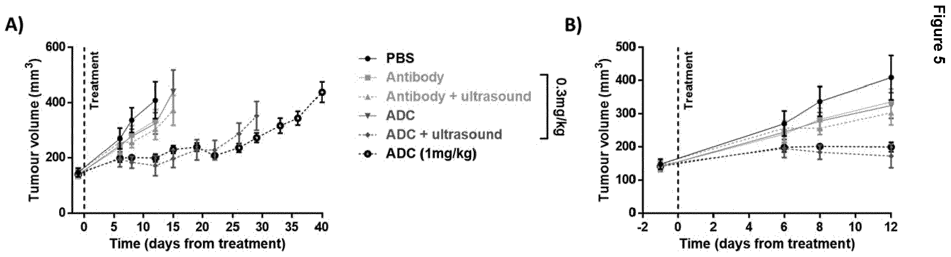

Tumours were measured twice a week after treatment until volume passed the maximum size given by the equation *(length* + *width)*/*2 > 12mm.* Figure 5A shows the average tumour growth for each group (each curve represents the average tumour growth for a specific group (± standard error of the mean) plotted until the first animal in that group was culled). This analysis indicates that groups treated with either a low dose of ADC (0.3mg/kg) + US or a higher dose of the same antibody (1mg/kg) showed reduced tumour growth compared to all other groups. Two-way ANOVA analysis performed on data until the first animal was culled confirmed that groups 5 and 6 were statistically different from the others at day 12 post treatment (Figure 5B).

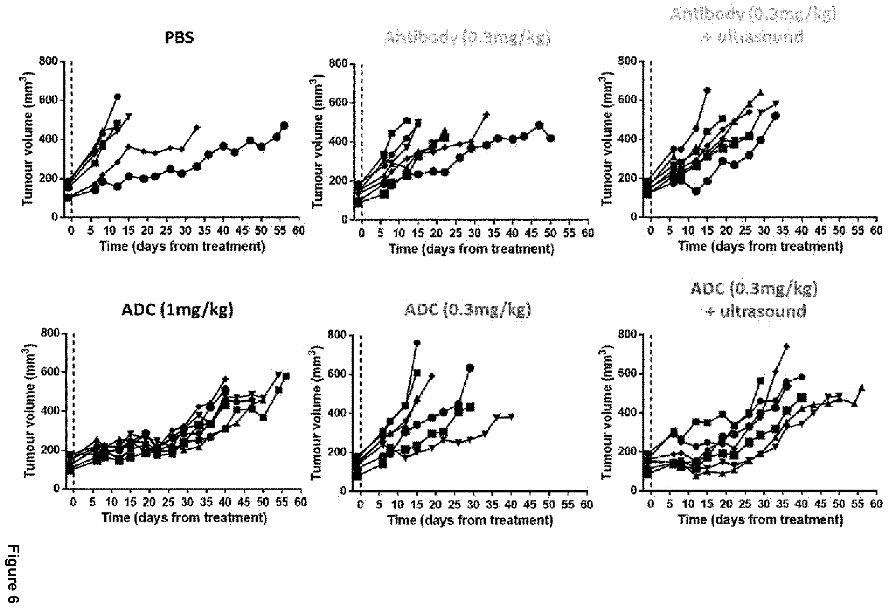

Individual tumour growth for every group is shown in Figure 6. Each curve represents a single tumour and is plotted until the value of (length + width)/2 > 12mm and the animal had to be culled.

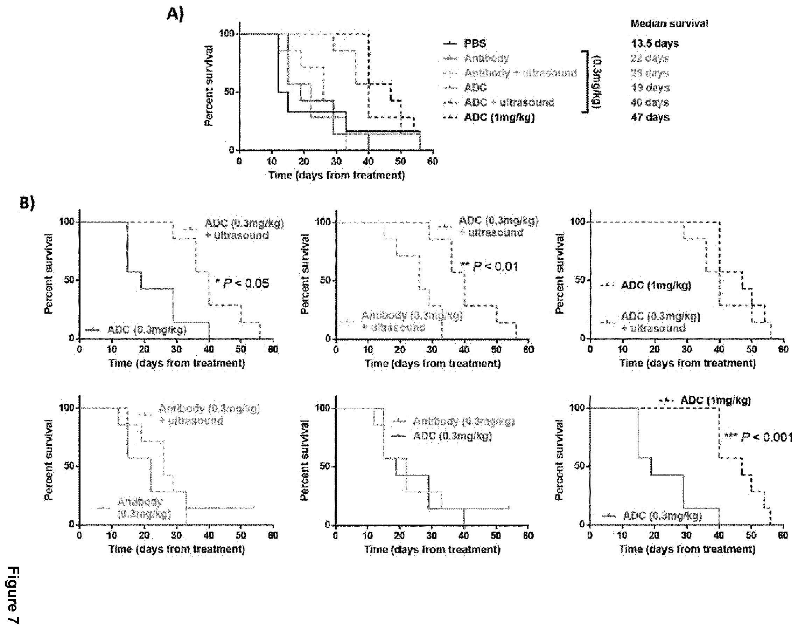

Although all animals died before the time limit of the experiment, survival analysis (Figure 7) confirmed what was observed in terms of tumour growth with groups 5 and 6 (ADC at 0.3mg/kg + US and ADC at 1mg/kg respectively) showing statistically significant longer survival than all the other groups. No difference between groups 1 to 4 was observed, or between groups 5 and 6. In Figure 7A, survival curves are shown for all groups with respective median survival. In Figure 7B, the survival graphs are for representative group pairs. Statistical significance was calculated by Log-Rank (Mantel-Cox) test.

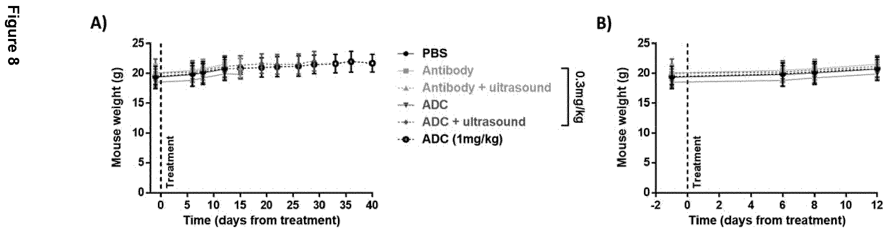

Mouse weight and appearance were monitored twice a week after treatment and no effects on welfare were observed for antibody and US treatment or their combination. Animal weight values over the course of the study are shown in Figure 8. Figure 8A shows average mouse weight for each group. Each curve represents the average weight for a specific group (± standard deviation) and is plotted until the first animal from that group has been culled. Figure 8B is as 8A but showing mouse weight only until the first animal dropped off the study (no statistically significant difference was found by 2-way ANOVA).

### Discussion

Analysis of the experimental data generated in this study indicates that control antibody administered with or without nanoparticle and US co-treatment did not significantly affect tumour growth and animal survival in comparison to the control group treated with PBS. This was the case also for the treatment with a low dose (0.3mg/kg) of ADC. However, treatment with the same dose of ADC in combination with nanoparticles and US treatment significantly reduced tumour growth ultimately leading to significantly extended animal survival. Both these effects were comparable to that achieved by treatment with the higher dose of ADC (1 mg/kg). No adverse effect was noticed in association with antibody and nanoparticle, or with US treatment, or their combination, and this is proved by the lack of difference in mouse weight between all treatment groups and the control one. This study demonstrates that administering ADC together with nanoparticle and application of ultrasound US treatment provides a significant therapeutic benefit, enabling lower doses of ADC to be administered whilst still maintaining efficacy.

## Claims

1. An antibody drug conjugate for use in a method of treating a solid tumour in a subject, which method comprises administering to the subject:
(i) a pharmaceutical composition comprising a plurality of nanoparticles, each nanoparticle comprising:
a cup having a cavity, wherein the cavity has an opening of at least 50nm in size, and
a gas pocket present in the cavity,
wherein the gas pocket is partially encapsulated by the cup, wherein the nanoparticle comprises a single nanocup having a single cavity, and wherein the pharmaceutical composition further comprises a pharmaceutically acceptable carrier or diluent, which is a liquid medium; and
(ii) the antibody drug conjugate, wherein the antibody drug conjugate comprises an anti-AXL antibody conjugated to a pyrrolobenzodiazepine dimer;
and exposing the subject to ultrasound.

2. The antibody drug conjugate for use according to claim 1 wherein the antibody comprises (i) an immunoglobulin heavy chain variable region having a CDR1 region with the amino acid sequence shown in SEQ ID NO: 3, a CDR2 region with the amino acid sequence shown in SEQ ID NO: 4, and a CDR3 region with the amino acid sequence shown in SEQ ID NO: 5; and (ii) an immunoglobulin light chain variable region having a CDR1 region with the amino acid sequence shown in SEQ ID NO: 6, a CDR2 region with the amino acid sequence shown in SEQ ID NO: 7, and a CDR3 region with the amino acid sequence shown in SEQ ID NO: 8.

3. The antibody drug conjugate for use according to claim 2 wherein the antibody comprises an immunoglobulin heavy chain variable region having the amino acid sequence shown in SEQ ID NO: 1.

4. The antibody drug conjugate for use according to claim 2 or claim 3 wherein the antibody comprises an immunoglobulin light chain variable region having the amino acid sequence shown in SEQ ID NO: 2.

5. The antibody drug conjugate for use according to any one of the preceding claims where the antibody drug conjugate is of formula (I):
Ab - L-D (I)
wherein:
Ab is an antibody that binds to AXL; and
L-D is a drug linker of formula (II): 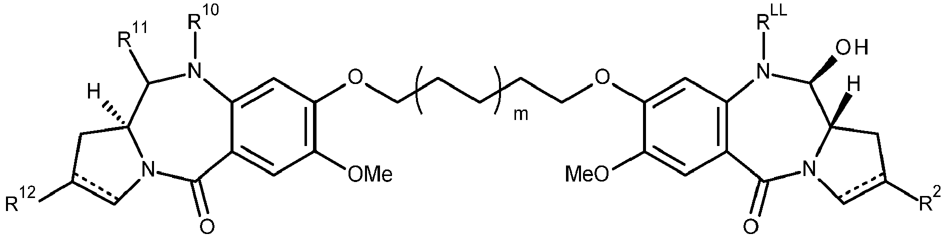 wherein:
(a) R^{LL} is a linker for connection to Ab;
(b) (i) R¹⁰ and R¹¹ together form a double bond between the nitrogen and carbon atoms to which they are attached; or (ii) R¹⁰ is R^{LLA} which is a linker for connection to Ab, and R¹¹ is OH; or (iii) R¹⁰ is a capping group R^{C}, and R¹¹ is OH;
(c) m is 0 or 1; and
(d) when there is a double bond between C2 and C3, R² is methyl;
when there is a single bond between C2 and C3, R² is either H or 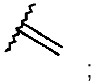
when there is a double bond between C2' and C3', R¹² is methyl;
when there is a single bond between C2' and C3', R¹² is H or

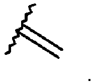

6. The antibody drug conjugate for use according to claim 5 , wherein R^{LL} is of formula (IIa): 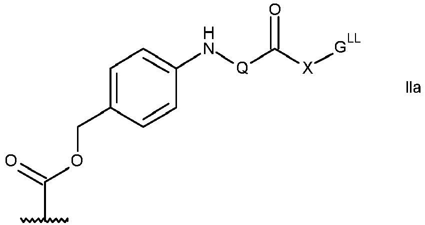 wherein:
Q is: 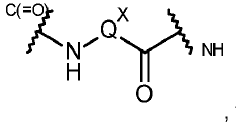 where Q^{x} is such that Q is an amino-acid residue, a dipeptide residue, a tripeptide residue or a tetrapeptide residue;
X is: 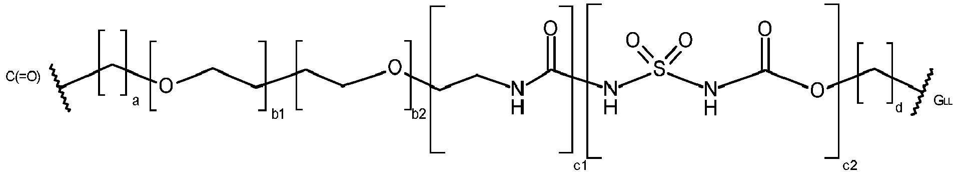
where a = 0 to 5, b1 = 0 to 16, b2 = 0 to 16, c1 = 0 or 1, c2 = 0 or 1, d = 0 to 5, wherein at least b1 or b2 = 0 and at least c1 or c2 = 0; and
G^{LL} is a linker group connected to the antibody.

7. The antibody drug conjugate for use according to any one of the preceding claims wherein the linker is a cleavable linker, such as a linker comprising a cathepsin cleavable sequence e.g. Val-Ala or Val-Cit.

8. The antibody drug conjugate for use according to any one of the preceding claims where the cytotoxin is conjugated to the antibody at an endogenous and/or engineered N-linked glycosylation site.

9. The antibody drug conjugate for use according to any one of claims 5 to 8 where L-D is compound B2.

10. The antibody drug conjugate for use according to any one of the preceding claims, wherein at least a part of the cavity of each nanoparticle is substantially part-spherical, preferably wherein the cavity of each nanoparticle is substantially part-spherical.

11. The antibody drug conjugate for use according to any one of the preceding claims, wherein the gas pocket is a bubble having a diameter of at least 50nm, preferably from 150 to 250nm.

12. The antibody drug conjugate for use according to any one of the preceding claims, wherein each nanoparticle is from 300 to 600nm in size.

13. The antibody drug conjugate for use according to any one of the preceding claims, wherein the nanoparticles are polymeric nanoparticles, preferably the nanoparticles comprise cross-linked polymer.

14. The antibody drug conjugate for use according to any one of the preceding claims, wherein the ultrasound has a pressure amplitude of from 1MPa to 4MPa.

15. A method of treating a solid tumour in a subject, which method comprises administering to the subject:
(i) a pharmaceutical composition comprising a plurality of nanoparticles, each nanoparticle comprising:
a cup having a cavity, wherein the cavity has an opening of at least 50nm in size, and
a gas pocket present in the cavity,
wherein the gas pocket is partially encapsulated by the cup, wherein the nanoparticle comprises a single nanocup having a single cavity, and wherein the pharmaceutical composition further comprises a pharmaceutically acceptable carrier or diluent, which is a liquid medium; and
(ii) an antibody drug conjugate comprising an anti-AXL antibody conjugated to a pyrrolobenzodiazepine dimer;
and exposing the subject to ultrasound.
